(19) Europäisches Patentamt
European Patent Office
Office européen des brevets

(11) Numéro de publication: **0 088 849**
**A1**

(12) **DEMANDE DE BREVET EUROPEEN**

(21) Numéro de dépôt: **82400441.0**

(22) Date de dépôt: **11.03.82**

(51) Int. Cl.$^3$: **C 07 D 207/09**
C 07 D 211/26, C 07 D 223/04
A 61 K 31/395

(43) Date de publication de la demande:
**21.09.83 Bulletin 83/38**

(84) Etats contractants désignés:
**AT BE CH DE FR GB IT LI LU NL SE**

(71) Demandeur: **CHOAY S.A.**
**48, Avenue Théophile-Gautier**
**F-75782 Paris Cédex 16(FR)**

(72) Inventeur: **Roger, Pierre**
**6, rue Paul Valéry**
**F-78423 Montigny Le Bretonneux(FR)**

(72) Inventeur: **Fournier, Jean-Paul**
**10, rue de Fontenay**
**F-78000 Versailles(FR)**

(72) Inventeur: **Choay, Patrick**
**7 Cour Jasmin**
**F-75016 Paris(FR)**

(74) Mandataire: **Gutmann, Ernest et al,**
**Cabinet Plasseraud 84, rue d'Amsterdam**
**F-75009 Paris(FR)**

(54) **Isomères lévogyres de composés benzènesulfonamides N-substitués, leur procédé de préparation et compositions pharmaceutiques les contenant.**

(57) L'invention a pour objet les isomères lévogyres de composés benzènesulfonamides N-substitués, leur procédé de préparation et leur application en tant que substance active de compositions pharmaceutiques les contenant.

L'invention concerne notamment des composés lévogyres de formule (VII) suivante:

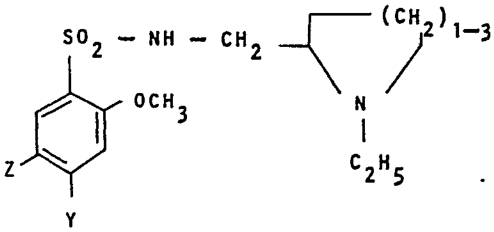

dans laquelle Y représente H, $NO_2$, $OCH_3$, $NH_2$ et Z peut représenter H, Cl, Br, $NO_2$.

Application à la préparation de compositions pharmaceutiques présentant notamment des propriétés anti-émétiques et anti-ulcéreuses.

EP 0 088 849 A1

# ISOMERES LEVOGYRES DE COMPOSES BENZENESULFONAMIDES N-SUBSTITUES, LEUR PROCEDE DE PREPARATION ET COMPOSITIONS PHARMACEUTIQUES LES CONTENANT

L'invention a pour objet de nouveaux composés présentant une structure de base du type benzènesulfonamides N-substitués.

L'invention est également relative aux procédés de préparation de ces composés.

L'invention vise également les compositions pharmaceutiques présentant notamment des propriétés anti-émétiques et anti-ulcéreuses et qui contiennent à titre de substance active, ces composés du type benzènesulfonamides N-substitués ou leurs sels obtenus avec des acides organiques ou minéraux physiologiquement acceptables, en association avec un véhicule pharmaceutique.

L'invention concerne des composés caractérisés en ce qu'ils répondent à la formule (I) suivante :

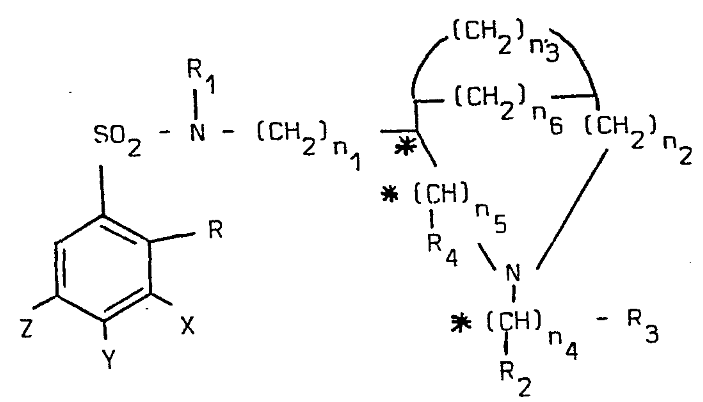

(I)

dans laquelle :

- $n_1$ varie de 0 à 3,
- $n_2$ peut varier de 1 à 4, et peut prendre la valeur 0, lorsque $n_5$ est différent de 0,
- $n_3$ prend les valeurs 0, 3, 4, 5 ou 6,
- $n_4$ varie de 0 à 4,
- $n_5$ varie de 0 à 2,
- $n_6$ varie de 0 à 2,
- R représente un radical alcoxy de 1 à 6 atomes de carbone, de préférence méthoxy ou éthoxy, ou un radical alcényloxy de 2 à 6 atomes de carbone, de

préférence de 3 atomes de carbone ;

- $R_1$ représente un atome d'hydrogène, un radical alcoyle linéaire ou ramifié de 1 à 6 atomes de carbone, un radical cycloalcoyle de 3 à 6 atomes de carbone, un radical alcényle de 2 à 6 atomes de carbone, de préférence de 3 à 6 atomes de carbone, un radical cycloalcényle de 3 à 6 atomes de carbone, de préférence un radical cyclopentène ou cyclohexène, un radical benzyle substitué ou non :
  - par un atome d'halogène, de préférence le fluor ;
  - par un radical alcoxy inférieur de 1 à 4 atomes de carbone ;
  - par un radical alcoyle linéaire ou ramifié de 1 à 4 atomes de carbone ;
- $R_2$ et $R_3$ représentent, indépendamment l'un de l'autre, un atome d'hydrogène, un radical alcényle de 2 à 6 atomes de carbone, de préférence de 3 à 6 atomes de carbone, un radical cycloalcényle de 3 à 6 atomes de carbone, de préférence un radical cyclopentène ou cyclohexène, un radical phényle substitué ou non :
  - par un atome d'halogène, de préférence le fluor ;
  - par un radical alcoxy inférieur de 1 à 4 atomes de carbone ;
  - par un radical alcoyle linéaire ou ramifié de 1 à 4 atomes de carbone ;
- $R_4$ représente un atome d'hydrogène, un radical alcoyle de 1 à 6 atomes de carbone ou un radical alcényle de 2 à 6 atomes de carbone ;
- X représente un atome d'hydrogène, un halogène, le groupe $OCH_3$, $CF_3$, $NO_2$ ou $NH_2$ ;
- Y représente un atome d'hydrogène, un halogène, un radical acylamino de 2 à 4 atomes de carbone, le groupe $OCH_3$, $CF_3$, $NO_2$ ou $NH_2$ ;
- Z représente un hydrogène, un halogène, le groupe $OCH_3$, $NO_2$, $NH_2$, $CF_3$, CN, $-\underset{\underset{O}{\|}}{C}-R_5$, $R_5$ étant un radical alcoyle

de 1 à 6 atomes de carbone, notamment éthyle ou méthyle ;

- $SR_6$, $R_6$ représentant un radical alcoyle linéaire ou ramifié ayant de 1 à 4 atomes de carbone, notamment un radical méthyle ou éthyle, $SO_2CH_3$, $SO_2C_2H_5$, $SO_{2n}C_3H_7$, $SO_{2i}C_3H_7$, $SO_2N\begin{matrix} R_7 \\ R_8 \end{matrix}$, $R_7$ et $R_8$ représentant, indépendamment l'un de l'autre, un atome d'hydrogène, un radical alcoyle de 1 à 6 atomes de carbone, et de préférence un hydrogène ou un méthyle.

Les astérisques apparaissant dans la formule (I) et les formules qui suivent, situent les carbones asymétriques ou susceptibles de l'être.

L'invention concerne également les isomères optiques dextrogyres et lévogyres, les diastéréoisomères et les mélanges racémiques des composés de formule (I) ainsi que les sels obtenus avec des acides organiques ou minéraux physiologiquement acceptables de ces isomères optiques, diastéréoisomères et racémiques.

A titre de sels organiques ou minéraux physiologiquement acceptables, on peut citer par exemple le chlorhydrate, le bromhydrate, les sulfates, les phosphates, le méthane sulfonate, l'acétate, le fumarate, le succinate, le lactate, le pyruvate, le citrate, le tartrate, le maléate, le malonate, le benzoate, le salicylate, le dichloro-2,6-benzoate, le chromoglycate, le benzène sulfonate, le dipropyl acétate, le glucose-1 phosphate.

Ne font pas partie de l'invention, les racémiques correspondant aux composés de formule suivante :

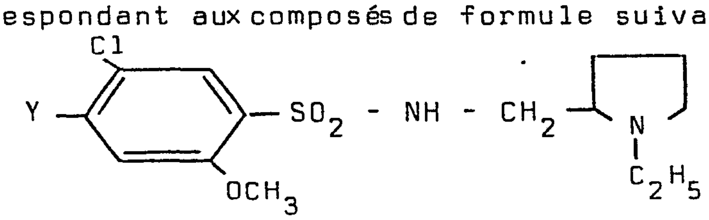

dans laquelle Y représente $NH_2$ ou $NO_2$, ainsi que leurs sels physiologiquement acceptables, car ils ont déjà fait l'objet du brevet français n° 2 386 524.

Les isomères optiques de ces composés ainsi

que leurs sels physiologiquement acceptables font néanmoins partie de l'invention.

Un groupe préféré de composés conformes à l'invention est constitué par les racémiques des composés de formule (I), ainsi que leurs sels physiologiquement acceptables.

A l'intérieur du groupe constitué par les racémiques, une classe préférée de composés conformes à l'invention est constituée par ceux de formule (II) :

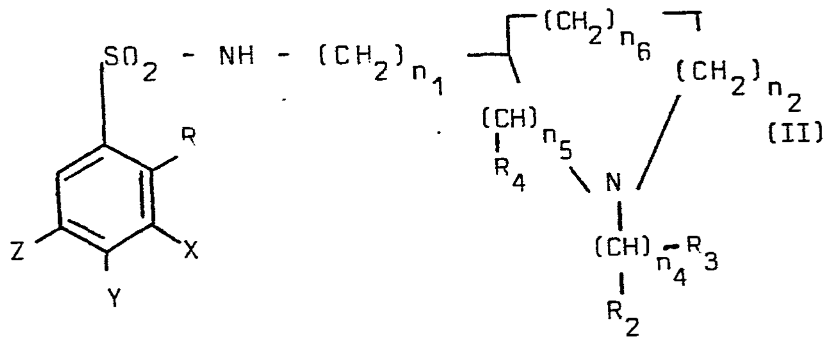

(II)

dans laquelle $n_1$, $n_2$, $n_4$, $n_5$, $n_6$, R, $R_2$, $R_3$, $R_4$, X, Y, Z ont les significations ci-dessus indiquées.

Ces composés se distinguent essentiellement de ceux de formule (I) par le fait que $n_3$ vaut 0.

A l'intérieur du groupe de composés racémiques répondant à la formule (II), une classe préférée de composés racémiques selon l'invention est constituée par ceux de formule (III) suivante :

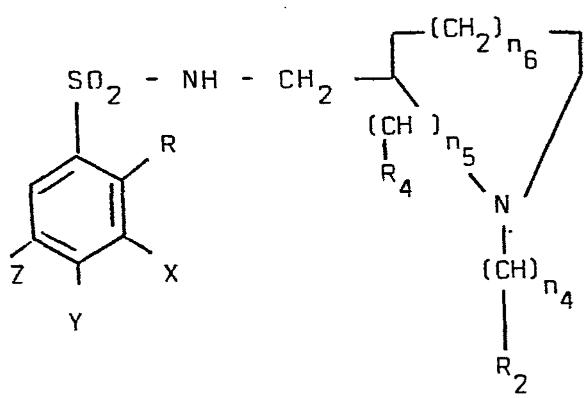

(III)

dans laquelle $n_4$, $n_5$, $n_6$, R, $R_2$, $R_3$, $R_4$, $R_5$, X, Y et Z ont les significations ci-dessus indiquées.

Ces composés se distinguent essentiellement

de ceux de formule (II) par le fait que $n_1$ et $n_2$ valent 1.

Parmi les composés de formule (III), une classe préférée de composés racémiques selon l'invention est constituée par ceux de formule (IV) suivante :

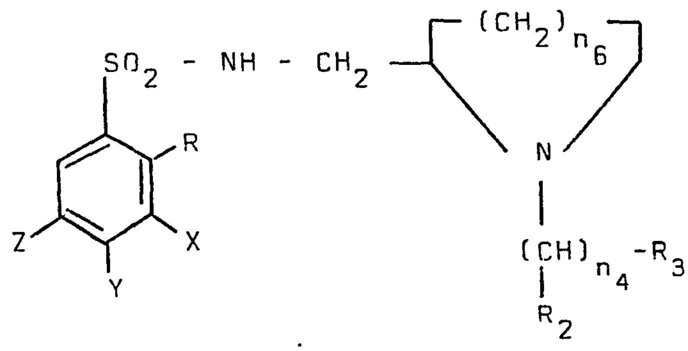

(IV)

dans laquelle $n_4$, $n_6$, R, $R_2$, $R_3$, X, Y et Z ont les significations ci-dessus indiquées.

Ces composés diffèrent des composés de formule (III) par le fait que $n_5$ vaut 0.

Parmi les composés de formule (IV), une classe préférée de composés racémiques selon l'invention est constituée par ceux de formule (V) :

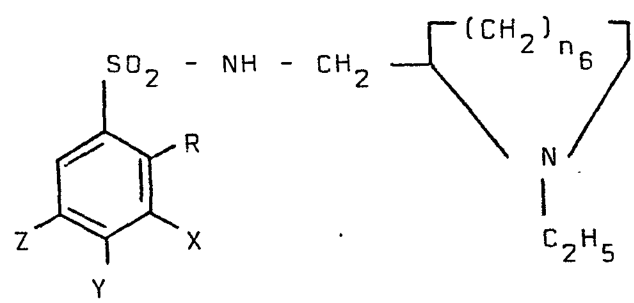

(V)

dans laquelle $n_6$, R, X, Y, Z ont les significations ci-dessus indiquées.

Ces composés se distinguent essentiellement des composés de formule (IV) par le fait que l'atome d'azote de l'hétérocycle est substitué par $C_2H_5$.

Parmi les composés de formule (V), une classe préférée de composés racémiques conformes à l'invention est constituée par ceux dans lesquels R représente

un groupe alcoxy, ayant de 1 à 6 atomes de carbone, de préférence méthoxy.

Une autre classe préférée de composés racémiques conformes à l'invention est constituée par ceux de formule (V) dans laquelle :

- R représente un groupe alcoxy ayant de 1 à 6 atomes de carbone, de préférence méthoxy, et
- X représente un atome d'hydrogène.

Parmi les composés de formule (V), une autre classe préférée de composés racémiques conformes à l'invention est constituée par ceux de formule (VI) suivante :

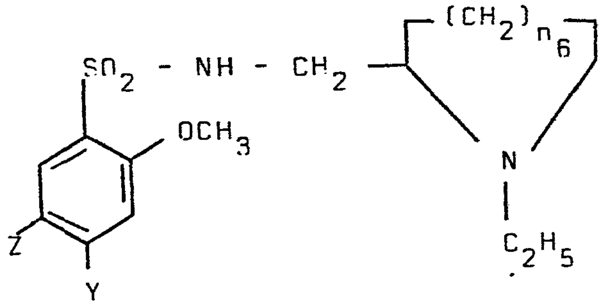

(VI)

dans laquelle :

- $n_6$ varie de 0 à 2,
- Y est choisi dans le groupe constitué par H, $NH_2$, $NO_2$, $OCH_3$,
- Z est choisi dans le groupe constitué par H, Cl, Br, $NO_2$, $OCH_3$, $SO_2CH_3$, $SO_2C_2H_5$, $SO_{2n}C_3H_7$, $SO_{2i}C_3H_7$.

Parmi les composés de formule (VI), une classe particulière préférée de composés selon l'invention est constituée par ceux de formule (VII) suivante :

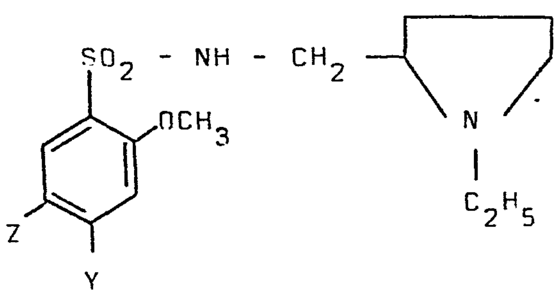

(VII)

Ces composés se distinguent des composés de formule (VI) par le fait que $n_6$ est égal à 0.

Une autre classe préférée de composés racémiques conformes à l'invention est constituée par les composés de formule (VII) dans laquelle Y est choisi dans le groupe constitué par H, $NO_2$, $OCH_3$, $NH_2$ et Z est choisi dans le groupe constitué par H, Cl, Br, $NO_2$, $OCH_3$, $SO_2CH_3$, $SO_2C_2H_5$, $SO_{2n}C_3H_7$, $SO_{2i}C_3H_7$, étant rappelé que Y est différent de $NH_2$ et de $NO_2$ quand Z représente le chlore.

Parmi ces composés, une classe préférée de composés racémiques selon l'invention est constituée par ceux dont le noyau aromatique est disubstitué en 2,4 par des groupes $OCH_3$, et dans lesquels Z est choisi dans le groupe constitué par Cl, Br, $OCH_3$, $SO_2CH_3$, $SO_2C_2H_5$, $SO_{2n}C_3H_7$, $SO_{2i}C_3H_7$.

A l'intérieur du groupe de composés racémiques répondant à la formule (II), un autre groupe préférée de composés racémiques conformes à l'invention est constitué par ceux de formule (VIII) suivante :

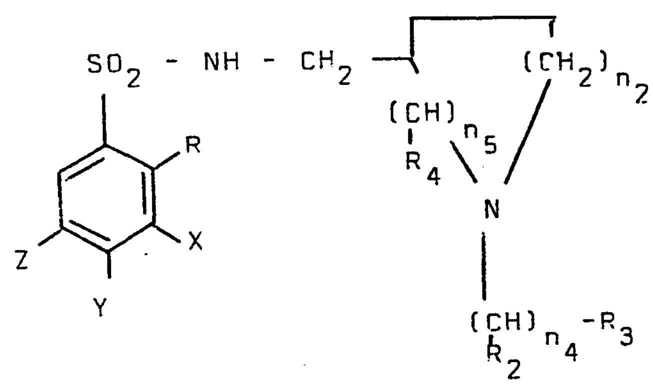

(VIII)

dans laquelle $n_4$, R, $R_2$, $R_3$, $R_4$, X, Y, Z ont les significations indiquées ci-dessus et $n_5$ vaut 1 ou 2, avec la condition que $n_2$ vaut 1 lorsque $n_5$ vaut 1 et $n_2$ vaut 0 lorsque $n_5$ vaut 2.

Parmi ces composés de formule (VIII), une classe préférée de composés racémiques conformes à l'invention est constituée par ceux de formule (IX) suivante :

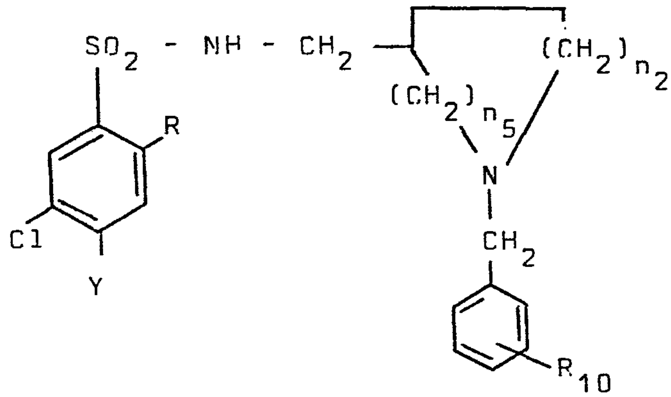

(IX)

dans laquelle $n_2$ et $n_5$ ont les significations ci-dessus indiquées, R représente un alcoxy de 1 à 6 atomes de carbone, notamment méthoxy, Y représente $NO_2$, $NH_2$ ou $OCH_3$, $R_{10}$ occupe l'une quelconque des positions 2 à 6 sur le noyau, notamment la position 4 et représente un atome d'halogène, notamment le chlore, un radical alcoxy inférieur de 1 à 4 atomes de carbone, un radical alcoyle de 1 à 4 atomes de carbone.

Des composés racémiques particulièrement préférés selon l'invention répondent aux formules suivantes :

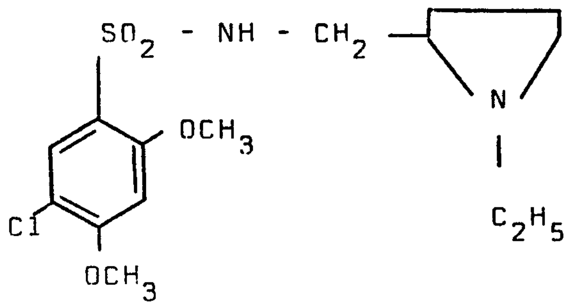

, HCl

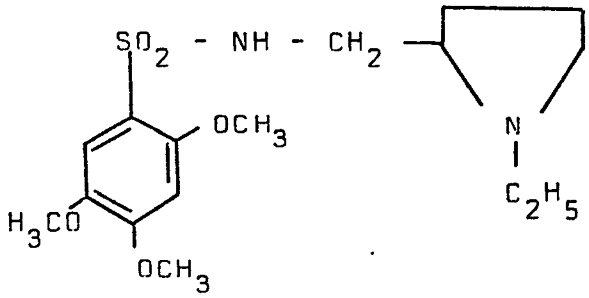

, HCl

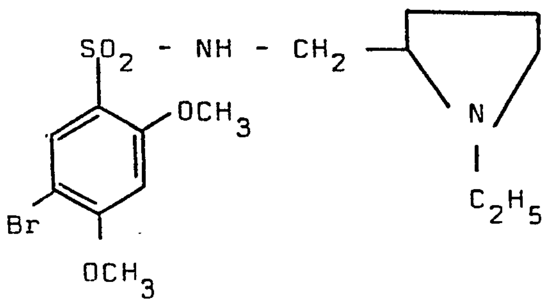

, HCl

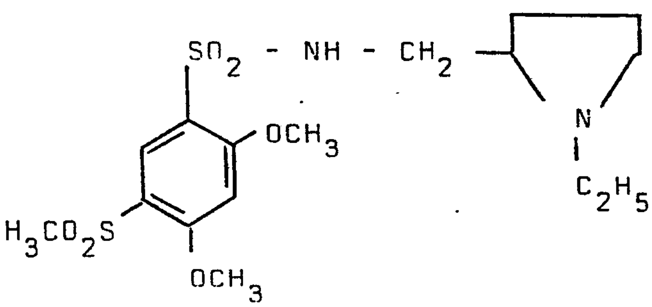

, HCl

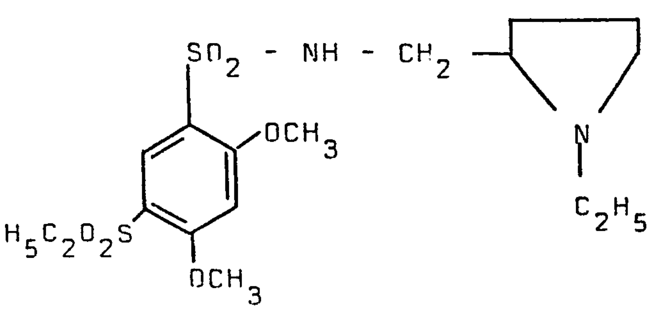

, HCl

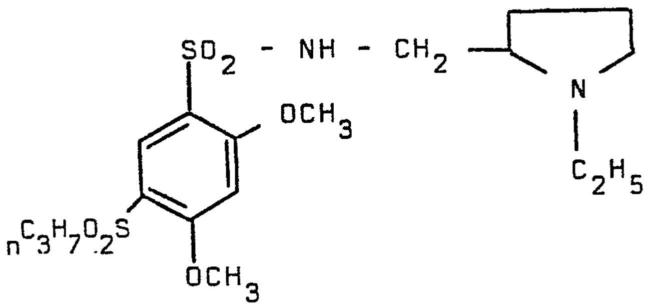

, HCl

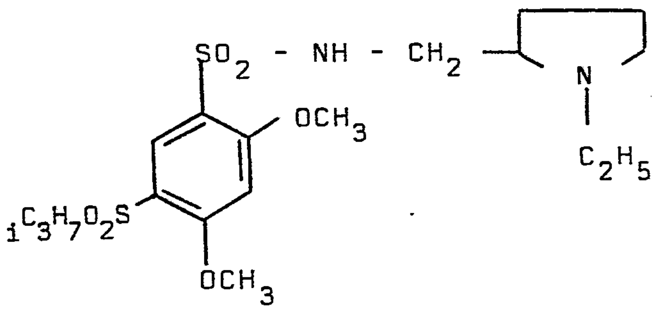

, HCl

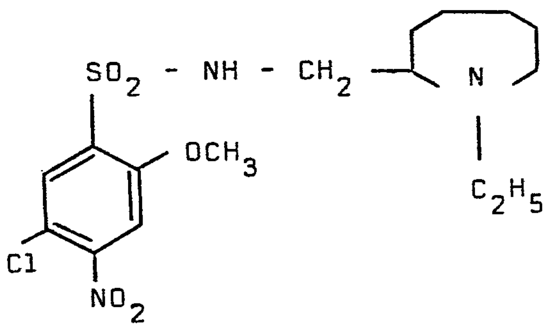

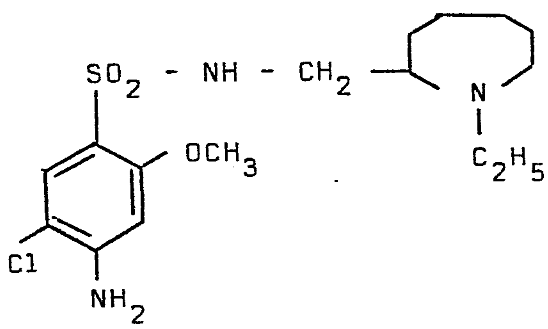

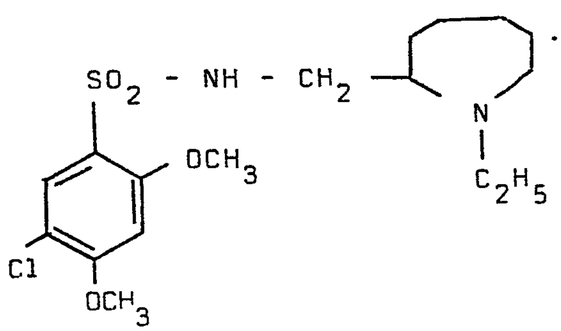

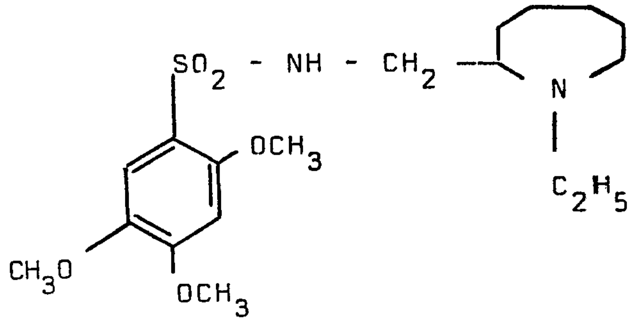

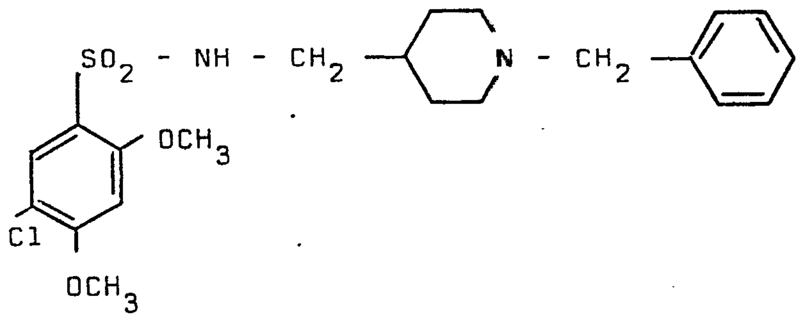

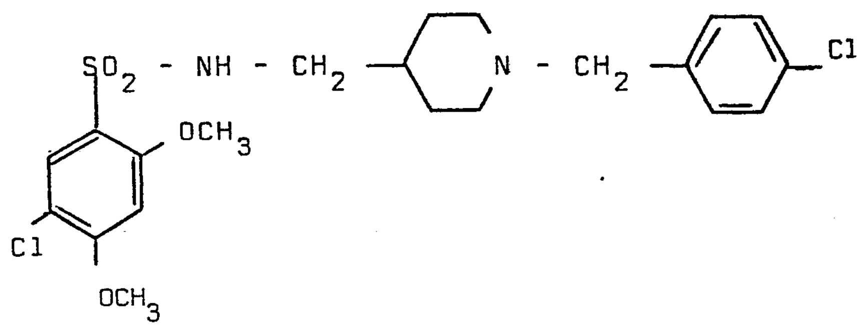

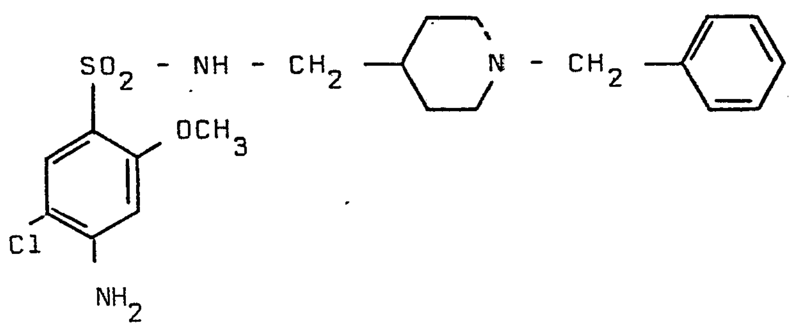

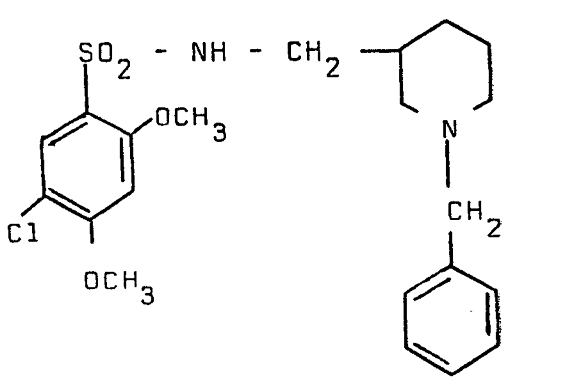

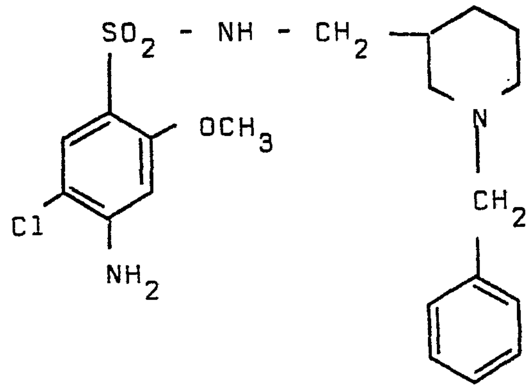

Parmi les composés de formule (I), une autre classe préférée de composés selon l'invention est constituée par les isomères optiques lévogyres ou dextrogyres.

Une classe particulièrement préférée de composés selon l'invention est constituée par les isomères optiques lévogyres.

Cet ensemble de composés comprend, d'une part, les isomères lévogyres obtenus à partir des racémiques précédemment définis, d'autre part les isomères lévogyres définis dans la suite.

Une classe préférée de composés lévogyres selon l'invention est constituée par ceux de formule (X) suivante :

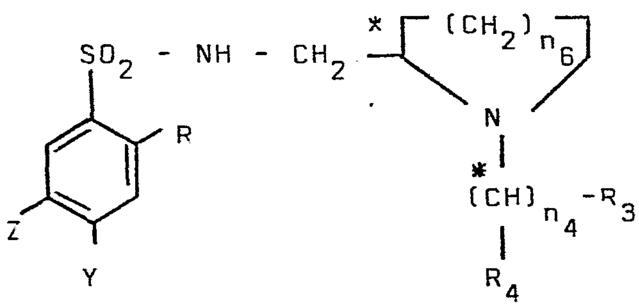

(X)

dans laquelle Y et Z ont les significations ci-dessus indiquées, R représente un groupe alcoxy de 1 à 6 atomes de carbone, notamment méthoxy ou éthoxy, $n_6$ peut varier de 0 à 2 et $\underset{R_4}{(\overset{|}{CH})}_{n_4}-R_3$ représente l'un des radicaux suivants :

$C_2H_5$, 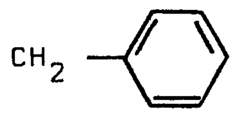 ou 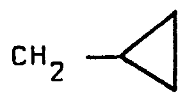

Parmi ce groupe de composés, un groupe particulièrement préféré de composés lévogyres selon l'invention est constitué par ceux de formule suivante (XI) :

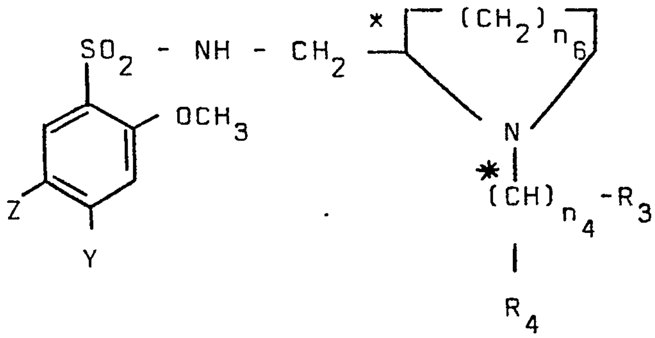

(XI)

dans laquelle $n_6$, $\underset{R_4}{\underset{|}{(CH)}}_{n_4}-R_3$ ont les significations ci-dessus indiquées, Y représente $OCH_3$, $NO_2$, $NH_2$ et Z représente Cl, $OCH_3$, $(CH_3)_2CH-SO_2$.

Parmi ces composés de formule (XI), un groupe de composés lévogyres conformes à l'invention est constitué par ceux de formule suivante (XII) :

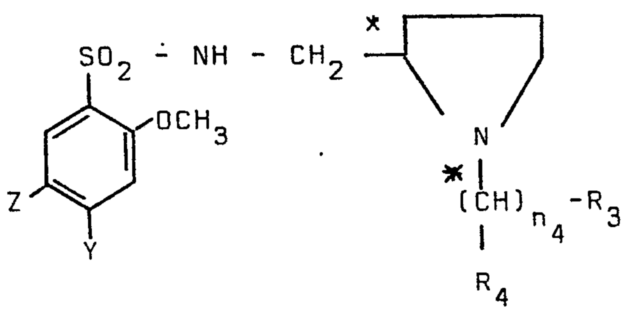

(XII)

dans laquelle $n_4$, $R_3$, $R_4$, Y et Z ont les significations indiquées ci-dessus.

Parmi ces composés de formule (XII), une classe avantageuse de composés lévogyres selon l'invention est constituée par ceux répondant à la formule (XIII) suivante :

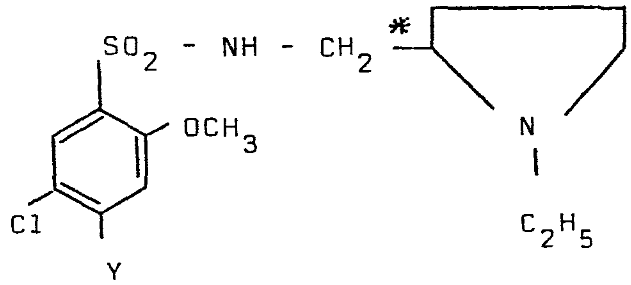

(XIII)

dans laquelle Y a les significations ci-dessus indiquées, et représente de préférence $OCH_3$, $NO_2$, $NH_2$.

Les composés lévogyres conformes à l'invention sont avantageusement sous forme de sels physiologiquement acceptables notamment chlorhydrate.

Les composés lévogyres particulièrement préférés de l'invention sont ceux de formule suivante :

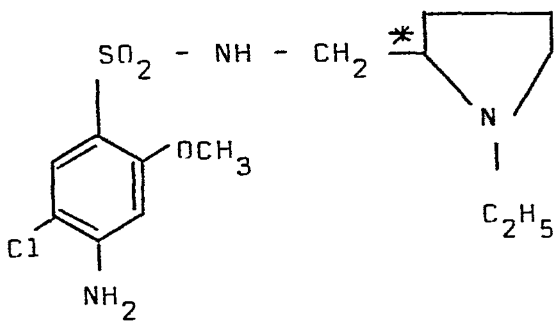

, HCl

composé n° 1 135

isomère S

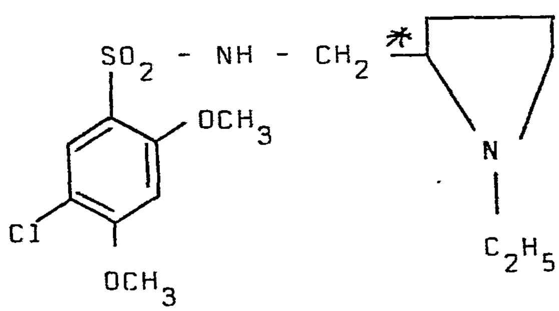

composé n° 1 138

isomère S

PREPARATION DES COMPOSES SELON L'INVENTION

Obtention des racémiques

Pour former les composés racémiques de formule (I) selon l'invention, on peut avoir recours à la réaction de sulfohalogénures sur des amines comme indiqué ci-après.

On peut utiliser des sulfohalogénures, notamment des sulfochlorures de formule :

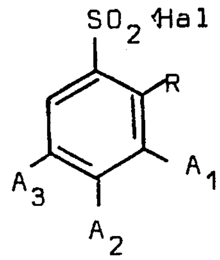

(XIV)

dans laquelle :

- Hal est un halogène, notamment brome ou de préférence chlore ;
- R représente un radical alcoxy de 1 à 6 atomes de carbone, de préférence méthoxy ou éthoxy, ou un radical alcényloxy de 2 à 6 atomes de carbone, de préférence de 3 atomes de carbone ;
- $A_1$, $A_2$, $A_3$ ont les significations indiquées pour X, Y et Z, à l'exception du groupe $NH_2$.

Les sulfochlorures de formule ci-dessous indiquée :

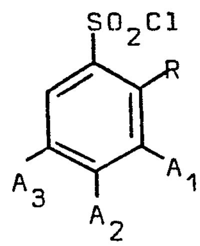

(XV)

dans laquelle R, $A_1$, $A_2$ et $A_3$ ont les significations ci-dessus indiquées, sont, pour certains d'entre eux, disponibles dans le commerce.

Lorsqu'ils ne sont pas disponibles dans le commerce, on peut les synthétiser soit par des méthodes classiques, soit pour certains d'entre eux par les méthodes exposées ci-après. Il s'agit notamment des sulfohalogénures de formule :

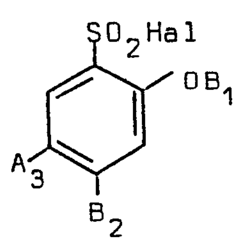

(XVI)

dans laquelle :

- Hal est un halogène,

- $B_1$ représente un radical alcoyle présentant de 1 à 4 atomes de carbone, de préférence méthyle,
- $B_2$ représente $NO_2$ ou un radical alcoxyle présentant de 1 à 4 atomes de carbone, de préférence méthoxy,
- $A_3$ a la même signification que Z, à l'exception de $NH_2$

Les méthodes dont question ci-après, se rapportent à la fabrication des sulfohalogénures de formule (XVII) :

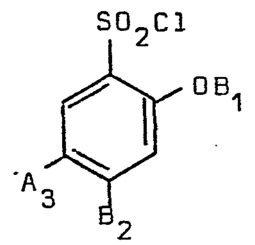

(XVII)

qui se différencie de la formule (XVI) par le fait que Hal représente le chlore, étant entendu que ces méthodes sont directement transposables aux autres sulfohalogénures.

Une première desdites méthodes utilise comme matière première une arylamine de formule :

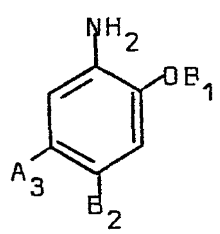

(XVIII)

dans laquelle $B_1$, $B_2$ et $A_3$ ont les significations ci-dessus indiquées, et à partir de laquelle :

a) on forme le sel de diazonium notamment le chlorure de diazonium de formule (XIX). Ce sel de diazonium est notamment obtenu en faisant réagir l'arylamine au sein d'une solution de l'acide halogéné correspondant, notamment d'acide chlorhydrique, avec une solution de nitrite d'un métal alcalin, et en maintenant le mélange réactionnel à une température de préférence inférieure à environ 10°C ;

b) on fait ensuite réagir le sel de diazonium obtenu en solution avec de l'anhydride sulfureux.

On opère de préférence en présence d'acide acétique ainsi que d'un catalyseur apte à contribuer à la transformation du groupe diazonium en groupe sulfohalogénure, notamment sulfochlorure. Ce catalyseur est, par exemple, à base de cuivre (réaction de Sandmeyer modifiée).

Les étapes a) et b) de cette réaction appliquées à titre d'exemple à la préparation des sulfochlorures de formule (XVII) dans laquelle Hal représente le chlore, peuvent être représentées comme suit :

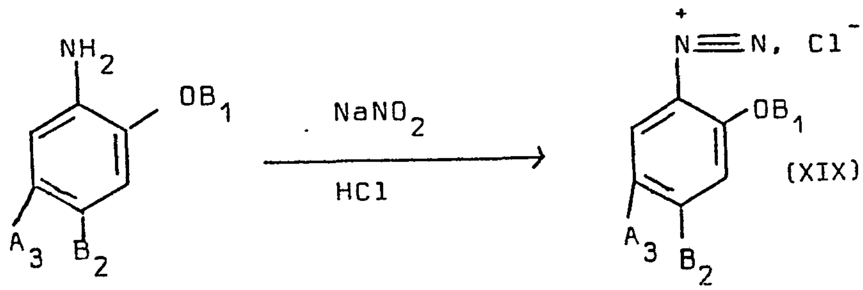


(XIX)

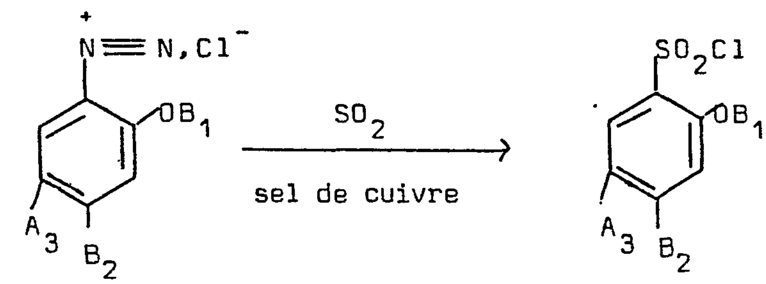


(XVII)

A titre d'arylamines préférées pour la préparation des sulfochlorures de formule (XVII), on peut citer :

- la diméthoxy-2,4 aniline,
- la chloro-5 diméthoxy-2,4 aniline,
- la bromo-5 diméthoxy-2,4 aniline,
- la triméthoxy-2,4,5 aniline,
- la diméthoxy-2,4 méthylsulfonyl-5 aniline,
- la diméthoxy-2,4 éthylsulfonyl-5 aniline,
- la diméthoxy-2,4 propylsulfonyl-5 aniline,
- la diméthoxy-2,4 isopropylsulfonyl-5 aniline,
- la chloro-5 méthoxy-2 nitro-4 aniline.

Une autre méthode pour préparer les sulfohalogénures de formule (XVI), notamment les sulfochlorures

de formule (XVII) dans lesquels $B_1$, $B_2$ et $A_3$ ont les significations ci-dessus indiquées, peut consister à procéder par sulfonation ou halogénosulfonation de préférence chlorosulfonation du composé de formule (XX) dans laquelle $B_1$, $B_2$, $A_3$ ont les significations sus-indiquées :

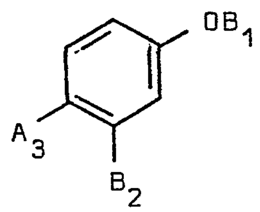

(XX)

Dans le cas de la sulfonation (par réaction du composé de formule (XX) avec de l'acide sulfurique) on obtient dans une première étape, l'acide sulfonique de formule (XXI) :

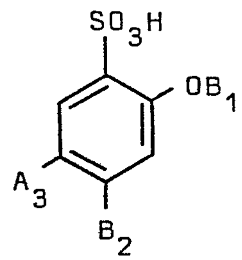

(XXI)

L'acide de formule (XXI) obtenu peut ensuite être transformé en sel d'une base organique ou minérale, par action de la base appropriée telle que l'hydroxyde de sodium, l'hydroxyde de potassium ou la pyridine.

Ce sel est représenté par la formule (XXII) suivante :

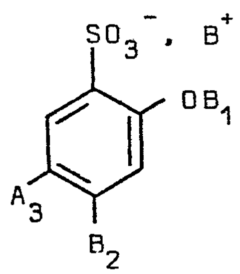

(XXII)

dans laquelle $B^+$ représente un métal, notamment alcalin, et $A_3$, $B_1$ et $B_2$ ont l'une quelconque des significations indiquées ci-dessus. Le sulfohalogénure de formule (XVI) notamment le chlorure de formule (XVII) est alors obtenu par action sur le composé de formule (XXI) -ou sur

un sel organique ou minéral correspondant de formule (XXII)- d'un agent chlorant, tel que le chlorure de thionyle, le pentachlorure de phosphore, ou l'oxychlorure de phosphore.

A titre d'exemple, le schéma réactionnel de l'obtention des composés de formule (XVII) dans laquelle $A_3$, $B_1$ et $B_2$ ont l'une quelconque des significations indiquées ci-dessus à partir des composés de formule (XX), peut se représenter comme suit :

(XX)

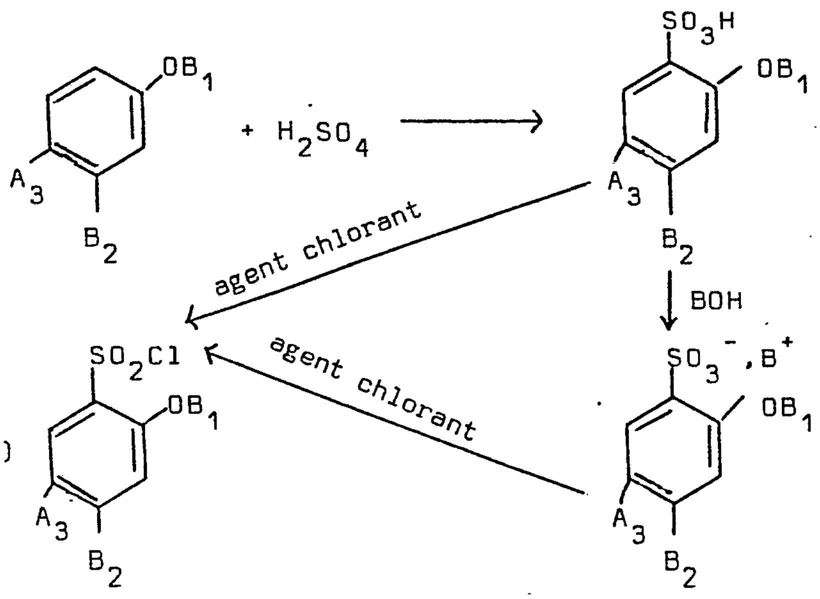


(XXI)

(XVII)

(XXII)

Dans le cas de la chlorosulfonation, on fait réagir l'acide chlorosulfonique sur le composé de formule (XX) dans laquelle $B_1$, $B_2$ et $A_3$ ont les significations ci-dessus indiquées.

La réaction peut s'écrire :

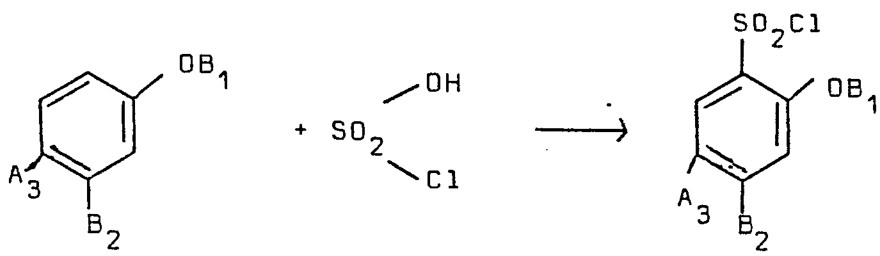

Pour l'obtention des composés racémiques de formule (I), on peut avoir recours à des amines de

formule :

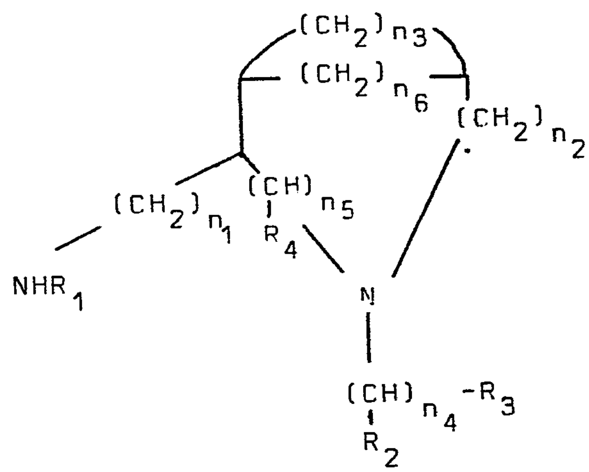

(XXIII)

dans laquelle $n_1$, $n_2$, $n_3$, $n_4$, $n_5$, $n_6$, $R_1$, $R_2$, $R_3$ et $R_4$ ont les significations ci-dessus indiquées.

A titre d'exemple, les amines de formule (XXIV) :

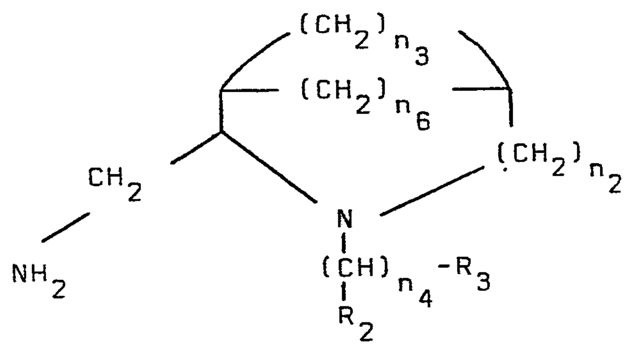

(XXIV)

peuvent être obtenues à partir des composés de formule (XXV) :

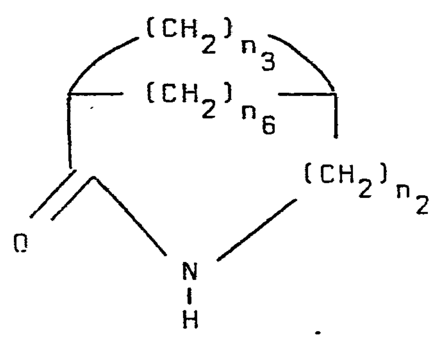

(XXV)

dans laquelle $n_2$, $n_3$ et $n_6$ ont les significations ci-dessus indiquées.

Ce composé de formule (XXV) est soumis à une alcoylation avec un halogénure d'alcoyle de formule :

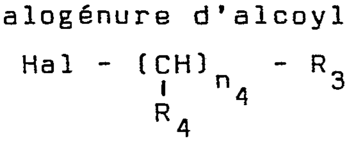

dans laquelle Hal représente un atome d'halogène, en présence d'une base forte telle que l'hydrure de sodium au sein d'un solvant tel que le xylène.

On obtient ainsi un composé de formule (XXVI) :

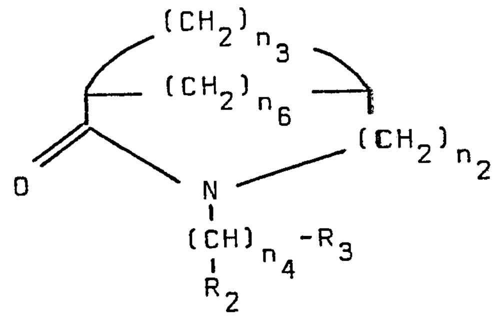

(XXVI)

dans laquelle $n_2$, $n_3$, $n_4$, $n_6$, $R_2$ et $R_3$ ont les significations indiquées ci-dessus.

Ce composé est ensuite traité par le sulfate de méthyle, le méthylate de sodium et le nitrométhane pour donner le composé de formule (XXVII) suivante :

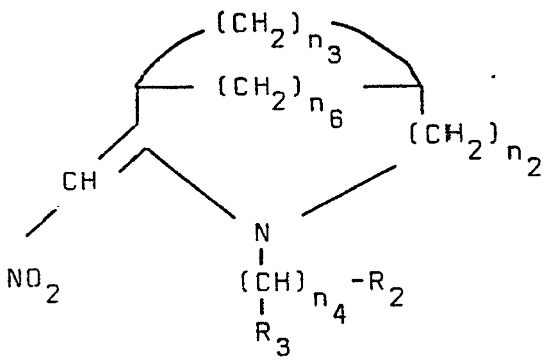

(XXVII)

dans laquelle $n_2$, $n_3$, $n_4$, $n_6$, $R_2$ et $R_3$ ont les significations indiquées ci-dessus.

Le groupe nitrométhylène du composé de formule (XXVII) est ensuite réduit par exemple à l'aide de nickel de Raney ou d'$AlLiH_4$. On obtient ainsi le composé de formule (XXIV) ci-dessus indiquée.

Un mode avantageux de préparation des composés racémiques selon l'invention de formule (IV) :

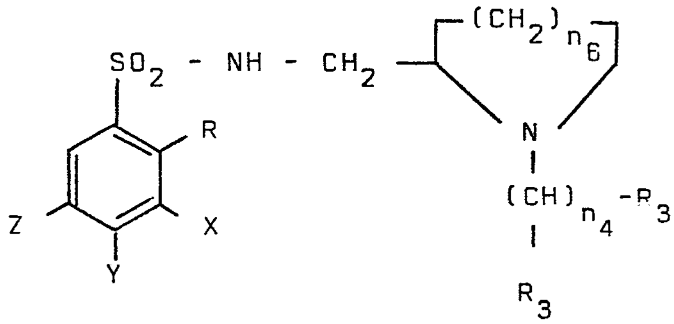

(IV)

dans laquelle $n_6$ vaut 0 à 2, de préférence 2, consiste à avoir recours aux amines de formule (XXVIII) :

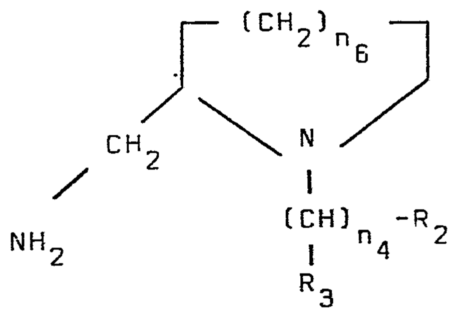

(XXVIII)

obtenues à partir des composés de formule (XXIX) :

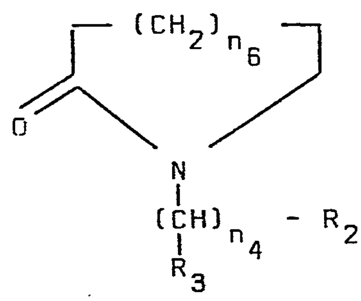

(XXIX)

selon le procédé indiqué ci-dessus.

Un mode avantageux de préparation des composés racémiques de l'invention de formule (VIII) :

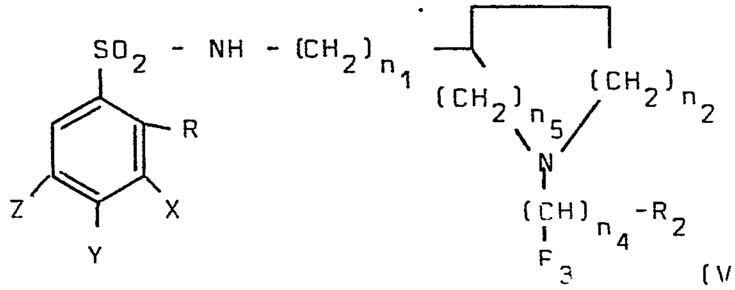

(VIII)

dans laquelle $n_5$ vaut 1 ou 2 et $n_2$ vaut respectivement 1 ou 0, et dans laquelle $R_1$, $R_2$, $R_3$, X,Y,Z, $n_1$ et $n_4$ ont les significations ci-dessus indiquées, consiste à utiliser les amines de formule (XXX) suivante :

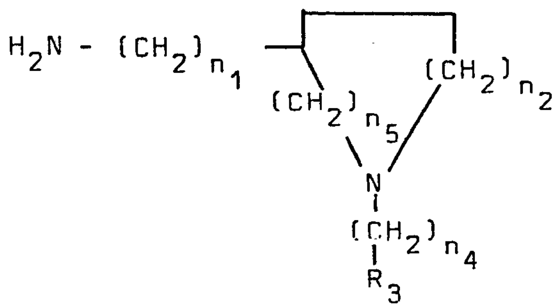

(XXX)

De façon pratique, on peut procéder comme dans l'exemple qui suit ou de façon équivalente.

Pour préparer des composés de formule (VIII) dans laquelle $n_2$ vaut 1 et $n_5$ vaut 1, on peut partir de l'amide de l'acide nipécotique substitué sur l'azote par $(\underset{R_2}{CH})_{n_4}-R_3$, $R_2$, $R_3$ et $n_4$ ayant les significations indiquées ci-dessus, que l'on réduit par $AlLiH_4$ selon le schéma réactionnel suivant :

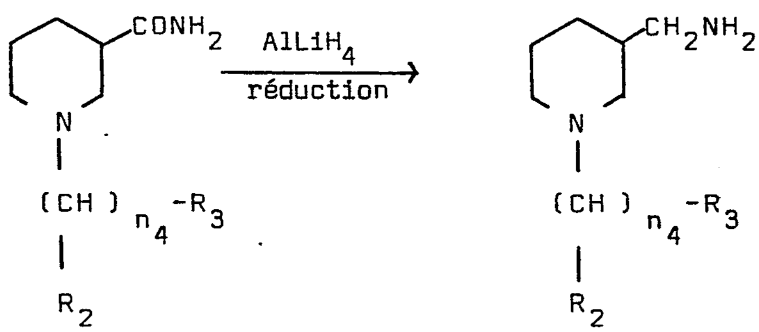


Pour préparer des composés de formule (VIII) dans laquelle $n_2$ vaut 0 et $n_5$ vaut 2, on peut partir de l'acide isonipécotique substitué sur l'azote par $(\underset{R_2}{CH})_{n_4}-R_3$, $R_2$, $R_3$ et $n_4$ ayant les significiations indiquées ci-dessus, que l'on réduit par exemple par $AlLiH_4$ selon le schéma réactionnel suivant :

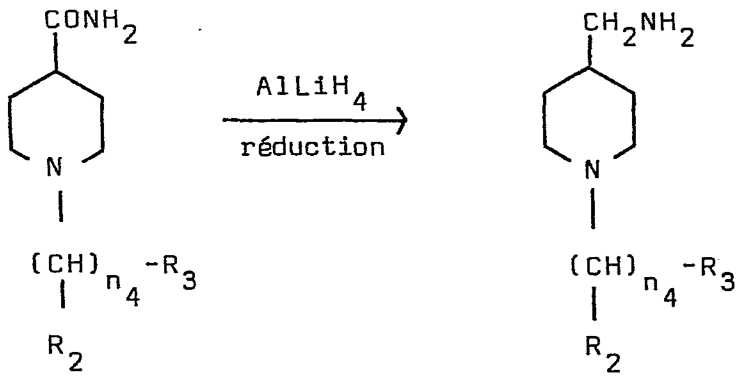


Un mode avantageux d'obtention des composés racémiques de l'invention de formule (XII) :

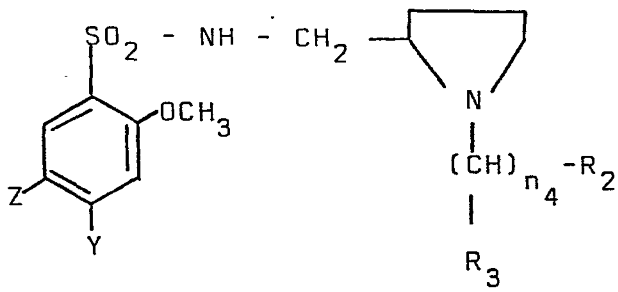

(XII)

consiste à avoir recours à l'amine de formule :

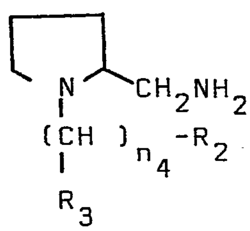

(XXXI)

Le composé de formule (XXXI) peut être obtenu à partir du composé de formule :

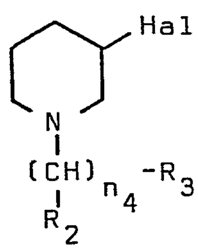

(XXXII)

dans laquelle $R_2$, $R_3$ et $n_4$ ont les significations ci-dessus indiquées, Hal représentant un halogène de préférence le chlore. On fait réagir ce composé de formule (XXXII) avec $C_6H_5-CH_2-NH_2$.

De façon pratique, la température du milieu réactionnel est généralement d'environ 65 à 75°C, et à la fin de la réaction, le milieu réactionnel est généralement porté à un pH d'environ 10.

Le composé obtenu a la formule suivante :

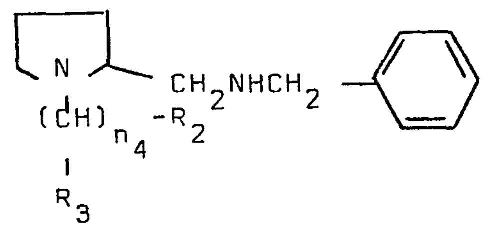

(XXXIII)

Le composé de formule (XXXIII) est ensuite soumis à une hydrogénolyse catalytique, par exemple en présence de charbon palladié, ce qui permet d'obtenir le composé de formule (XXXI).

Un mode avantageux d'obtention des composés racémiques selon l'invention, de formule (XXXIV) suivante :

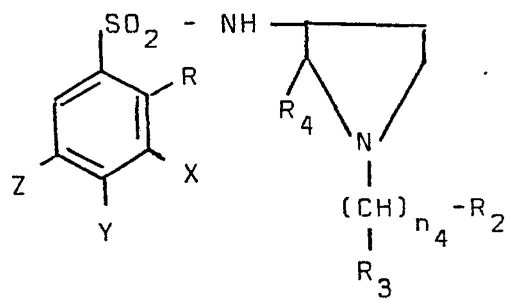

(XXXIV)

dans laquelle X, Y, Z, R, $R_2$, $R_3$ et $R_4$ ont les significations ci-dessus indiquées, consiste à avoir recours aux pyrrolidines de formule (XXXV) :

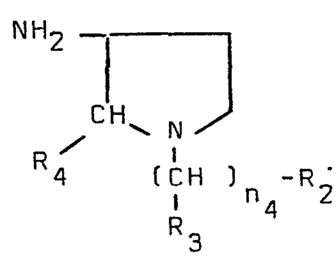

(XXXV)

Ces composés peuvent être préparés à partir des composés de formule (XXXVI) :

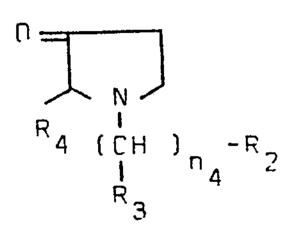

(XXXVI)

De façon pratique on peut hydrogéner le composé de formule (XXXVI) par exemple à l'aide de nickel de Raney en présence d'ammoniac et de méthanol selon la méthode décrite page 1 227 du Journal of Medicinal Chemistry, 1981, vol. 24, n° 10 et dans les demandes de brevet japonais publiées n° 5 328 161, 5 328 163 et 5 522 699.

L'obtention des racémiques de formule (I) peut être effectuée en condensant un sulfochlorure de formule (XV) :

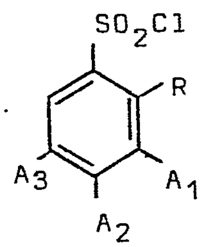

(XV)

sur une amine de formule :

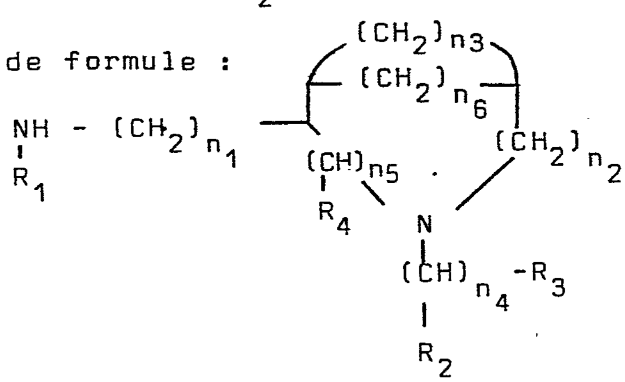

(XXIII)

ce qui conduit à l'obtention du composé de formule (I) sous forme de chlorhydrate dont la formule est :

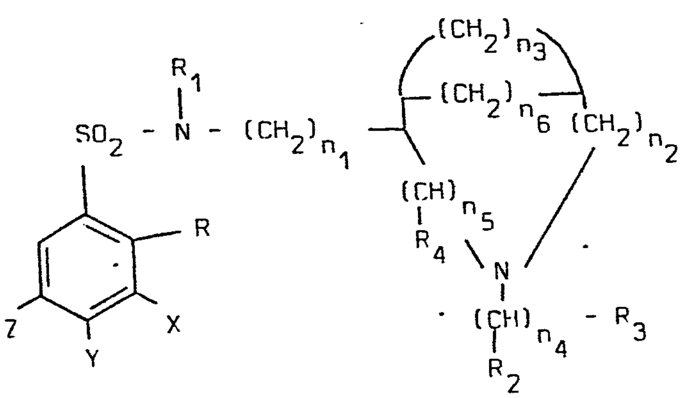

, HCl

(XXXVII)

étant entendu que si à la place du sulfochlorure de départ, on utilise un autre halogénure, on obtient le composé de formule (I) sous forme de l'halogénate

correspondant.

Le passage du chlorhydrate de formule ci-dessus indiquée au composé de formule (I), non salifié, c'est-à-dire sous forme de base, peut se faire en solution, par réaction avec une base forte telle que l'hydroxyde de sodium, ou de potassium, ou toute autre base présentant des caractères de basicité équivalents.

La transformation du chlorhydrate de formule (XXXVII) en un sel différent peut se faire en passant par la base de formule I), puis en salifiant celle-ci selon des procédés classiques.

L'obtention des composés racémiques de formule (I) dans laquelle l'un au moins des groupes X, Y, Z représente $NH_2$ est réalisée en réduisant le composé de formule (I) dans lequel l'un au moins des X, Y, Z représente $NO_2$ par hydrogénation catalytique ou par réduction chimique.

A titre d'amines préférées pour la préparation des racémiques de formule (I), on peut citer :

- l'aminométhyl-2 éthyl-1 pyrrolidine,
- l'aminométhyl-2 benzyl-1 pyrrolidine,
- l'aminométhyl-2-cyclopropyl-1 pyrrolidine,
- l'amino-méthyl-2 éthyl-1 azépine,
- l'aminométhyl-3 benzyl-1 pipéridine,
- l'aminométhyl-3 (chloro-4 benzyl)-1 pipéridine,
- l'aminométhyl-4 benzyl-1 pipéridine,
- l'aminométhyl-4 (chloro-4-benzyl)-1 pipéridine.

Obtention des isomères optiques selon l'invention, notamment des lévogyres

Deux voies d'accès sont possibles :

1° on peut partir du racémique d'un composé de formule (I) selon l'invention (obtenu par réaction du sulfochlorure de formule (XV) sur le racémique de l'amine de formule (XXIII) ou (XXIV) que l'on dédouble par exemple par l'acide tartrique.

Le procédé de dédoublement consiste à salifier le racémique par la quantité stoechiométrique d'acide tartrique optiquement actif. Les sels obtenus sont cristallisés plusieurs fois dans l'alcool, de préférence méthanol ou éthanol, jusqu'à l'obtention d'une pureté optique supérieure à 95 %. Par alcalinisation, les bases sont libérées et recristallisées. On obtient ainsi les composés lévogyre et dextrogyre correspondants.

2° On peut obtenir directement l'isomère optique lévogyre du composé de formule (I) (respectivement dextrogyre) en faisant réagir un sulfochlorure sur l'isomère optique lévogyre (respectivement dextrogyre) de l'amine appropriée.

Il est entendu que dans ce cas, on obtiendra le composé lévogyre (respectivement dextrogyre) sous forme de son chlorhydrate et que le passage du chlorhydrate au composé non salifié se fait de façon semblable à celle qui a été décrite à propos des racémiques.

De même, l'obtention des composés lévogyres (respectivement dextrogyres) de l'invention dans laquelle l'un au moins des X, Y ou Z représente $NH_2$, est effectuée de façon semblable à ce qui a été décrit à propos des composés racémiques.

A titre d'amines préférées utilisées, sous forme d'isomères optiques pour la préparation des composés lévogyres de formule (I) selon l'invention, on peut citer les isomères optiques des amines déjà citées à titre d'amines préférées dans la préparation des composés racémiques selon l'invention.

Les exemples suivants relatifs à la préparation d'un certain nombre de nouveaux benzènesulfonamides servent à illustrer l'invention, mais ne sont pas limitatifs.

On décrira d'abord des exemples de fabrication des matières premières à partir desquelles ont été obtenues les substances décrites dans les exemples.

Préparation du chlorure de triméthoxy-2,4,5-benzènesulfonyle

Elle peut être effectuée selon deux procédés.

1er procédé

Dans un tricol de 500 $cm^3$, muni d'un thermomètre, d'une ampoule à réactif, d'une garde à chlorure de calcium et d'un système d'agitation magnétique, sont placés 42 g (0,25 mole) de triméthoxy-1,2,4 benzène en solution dans 200 $cm^3$ de chloroforme pur. Au milieu réactionnel, placé sous atmosphère d'azote et refroidi vers -10°C, sont ajoutés goutte à goutte 80 $cm^3$ d'acide chlorosulfonique. Il se forme successivement un précipité laiteux blanc-crème, puis une solution verdâtre qui vire au brun. L'addition terminée, le milieu réactionnel est laissé en contact 1 h à température ambiante, puis est versé sur de la glace pilée. Le précipité obtenu est extrait par du chloroforme ; la phase organique est ensuite séchée sur sulfate de sodium puis évaporée sous pression réduite. Le résidu brun formé est lavé avec le minimum de toluène jusqu'à l'obtention d'un solide beige qui est ensuite chromatographié sur colonne de silice.

L'élution avec du benzène, puis avec un mélange benzène - chloroforme (50-50) donne le chlorure de triméthoxy-2,4,5 benzènesulfonyle.

Rdt 46 % F 147°C

RMN ($CDCl_3$) à 80 MHz :

δ 7,29 ppm (s 1H ArH) ; δ 6,55 ppm (s 1H ArH) ; δ 3,93, 3,78 et 3,53 ppm (3s 9H $OCH_3$)

IR (KBr) $\gamma$ $SO_2$, as 1350 $cm^{-1}$, s 1160 $cm^{-1}$.

2ème procédé

Il comporte 4 étapes :

Diméthoxy-3,4 chlorobenzène :

Dans un tricol d'un litre, muni d'un système d'agitation magnétique, d'un thermomètre, d'une garde à chlorure de calcium et d'une ampoule à réactif, sont introduits à froid vers 0°C et successivement 138 g (1 mole) de vératrole puis goutte à goutte 135 g (1 mole) de chlorure de sulfuryle. L'addition terminée, le milieu réactionnel est ramené à température ambiante, puis après contact une heure est

distillé sous pression réduite.

Rdt 83% Pf : 120°C

sous 1 999,83 Pa (15 mm Hg)

Dimethoxy-4,5 nitro-2 chlorobenzène :

Dans un tricol d'un litre, muni d'un système d'agitation magnétique, d'un thermomètre et d'une ampoule à réactif, sont introduits successivement 143,9 g (0,83 mole) de diméthoxy-3,4 chlorobenzène, puis goutte à goutte 166 $cm^3$ d'acide nitrique (d=1,38), sans que la température dépasse 25°C. L'addition terminée, le mélange réactionnel est laissé 1,5 h en contact puis filtré.

Rdt 95% F 105°C

Triméthoxy-2,4,5 nitrobenzène :

Dans un ballon de 2 litres, muni d'un réfrigérant, sont introduits successivement une solution de potasse méthanolique (100 g de KOH dans 500 $cm^3$ de méthanol), puis 100 g (0,46 mole) de diméthoxy-4,5 nitro-2 chlorobenzène et du carborandum. Le mélange est porté à l'ébullition au reflux 6 h. Après refroidissement, le milieu réactionnel est filtré ; le précipité obtenu est lavé avec du méthanol.

Rdt 95% F 129°C

Triméthoxy-2,4,5 aniline :

Dans un ballon de 500 $cm^3$, muni d'un réfrigérant, sont ajoutés successivement 21,3 g (0,1 mole) de triméthoxy-2,4,5 nitrobenzène, 80 g de chlorure stanneux chimiquement pur pour miroitiers, 100 $cm^3$ d'une solution d'acide chlorhydrique (d=1,18) et du carborandum. Le mélange est porté à l'ébullition à reflux 1 h. Après refroidissement, une solution de lessive de soude est ajoutée jusqu'à dissolution du précipité. La solution obtenue est extraite par du chlorure de méthylène. Les extraits organiques sont séchés sur sulfate de sodium puis évaporés sous pression réduite. Le résidu obtenu est cristallisé dans de l'éthanol.

Rdt 80% F 94°C

Chlorure de triméthoxy-2,4,5 benzènesulfonyle :

Dans un tricol de 250 $cm^3$, muni d'un système d'agitation et d'un thermomètre, sont introduits successivement 18,3 g (0,1 mole de triméthoxy-2,4,5 aniline puis 50 $cm^3$ d'une solution d'acide chlorhydrique (d=1,18). Après un contact de 4 heures, l'amine est diazotée à -5°C par addition d'une solution de nitrite de sodium (10 g de $NaNO_2$ dans 50 $cm^3$ d'eau). Le sel de diazonium obtenu est versé lentement dans un tricol, chauffé à 40°C et sous atmosphère d'azote, contenant 200 $cm^3$ d'acide acétique saturé en anhydride sulfureux et 7 g de chlorure cuivrique. Le mélange est porté 2 h à 60°C, puis est versé sur de la glace pilée.

Rdt 35%   F 147°C

PREPARATION DU CHLORURE DE CHLORO-5 DIMETHOXY-2,4 BENZENESULFONYLE

Elle est effectuée en 2 étapes à partir du diméthoxy-1,3 benzène :

<u>1ère étape</u> : <u>Obtention du diméthoxy-2,4 chlorobenzène</u>

Elle s'effectue en suivant la technique de G. CASTELFRANCHI et G. BORRA rapportée dans Annali di Chimica, 1953, <u>43</u>, 293.

Dans un tricol de 500 $cm^3$, muni d'un système d'agitation magnétique, d'un thermomètre, d'une garde à chlorure de calcium et d'une ampoule à réactif, et refroidi vers 10°C, sont introduits successivement 97,5 g (0,70 mole) de diméthoxy-1,3 benzène puis goutte à goutte 96,5 g (0,70 mole) de chlorure de sulfuryle. Une fois l'addition terminée, la solution est ramenée à la température ambiante et laissée en contact 2 h puis distillée.

Rdt 85%   PE : 137°C sous 2 399,80 Pa (18 mm Hg)

<u>2ème étape</u> : <u>Passage au chlorure de chloro-5 diméthoxy-2,4 benzènesulfonyle</u>

Dans un tricol de 500 $cm^3$, muni d'un système d'agitation magnétique, d'une garde à chlorure de calcium,

d'un thermomètre et d'une ampoule à réactif, sont introduits 35 g (0,2 mole) de diméthoxy-2,4 chlorobenzène en solution dans 250 $cm^3$ de chloroforme pur. La solution est refroidie vers 0°C, puis est additionnée goutte à goutte de 50 $cm^3$ (0,75 mole) d'acide chlorosulfonique. Une fois l'addition terminée, le milieu réactionnel est ramené à température ambiante, puis laissé en contact 3 h ; il est ensuite versé sur de la glace pilée. Le mélange obtenu est épuisé au chloroforme. Les extraits organiques sont séchés sur sulfate de sodium puis concentrés jusqu'à cristallisation. Le solide obtenu est recristallisé dans un mélange éther éthylique/benzène.

Rdt 74% F 175°C

RMN ($CDCl_3$) à 80 MHz :

$\delta$ 7,87 ppm (s 1H ArH) ; $\delta$ 6,55 ppm (s 1H ArH) ; $\delta$ 4 et 3,96 ppm (2s 6H $OCH_3$)

IR (KBr) : $\nu$ $SO_2$, as 1385 $cm^{-1}$, s 1170 $cm^{-1}$

PREPARATION DU CHLORURE DE BROMO-5 DIMETHOXY-2,4 BENZENE-SULFONYLE

Elle est effectuée en 2 étapes à partir du diméthoxy-1,3 benzène :

<u>1ère étape</u> : <u>Obtention du diméthoxy-2,4 bromobenzène</u>

Elle s'effectue en suivant le protocole de S.T. FENG et K.Y. CHIU rapportée dans Hsûeh Pao, 1959, <u>25</u>, 277.

Dans un tricol de 250 $cm^3$, muni d'un système d'agitation magnétique, d'un thermomètre, et refroidi vers 10°C, sont ajoutés successivement 27,6 g (0,2 mole) de diméthoxy-1,3 benzène puis par petites portions 36 g (0,2 mole) de <u>N</u>-bromosuccinimide. Une fois l'addition terminée, le milieu réactionnel est ramené à température ambiante et laissé en contact 2 h. Après lavage à l'eau et extraction au chloroforme, les extraits organiques sont séchés sur sulfate de sodium puis concentrés sous pression réduite. Le liquide

résiduel est distillé.

Rdt 82% PE : 150°C sous 1 999,83 Pa (15 mm Hg)

2ème étape : Passage au chlorure de bromo-5 diméthoxy-2,4 benzènesulfonyle

Il est obtenu selon le même protocole que celui décrit dans l'exemple 3 pour préparer le chlorure de chloro-5 diméthoxy-2,4 benzènesulfonyle. En utilisant 65,1 g (0,3 mole) de diméthoxy-2,4 bromobenzène et 100 $cm^3$ d'acide chlorosulfonique, le rendement est de 77%.

F 195°C

RMN ($CDCl_3$) à 60 MHz

δ 7,85 ppm (s 1H ArH) ; δ 6,70 ppm (s 1H ArH) ;

δ 3,92 et 3,88 ppm (2s 6H $OCH_3$) ;

IR (KBr) ; ν $SO_2$, as 1385 $cm^{-1}$, s 1165 $cm^{-1}$

CHLORURES D'ALKYLSULFONYL-5 DIMETHOXY-2,4 BENZENESULFONYLES

Ils sont obtenus à partir du chlorure de diméthoxy-2,4 benzènesulfonyle dont la préparation est rapportée par C. M. SUTER et H. L. HANSEN dans J. of Am. Chem. Soc. 1933, 55, 2 080. Le schéma général de leur préparation est le suivant :

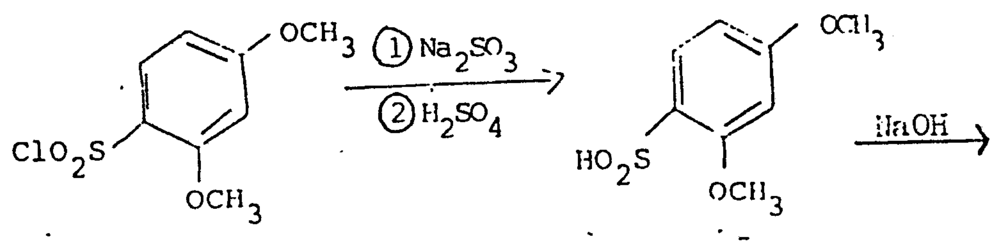


Chlorure de diméthoxy-2,4 benzènesulfonyle

Acide diméthoxy-2,4 benzènesulfinique

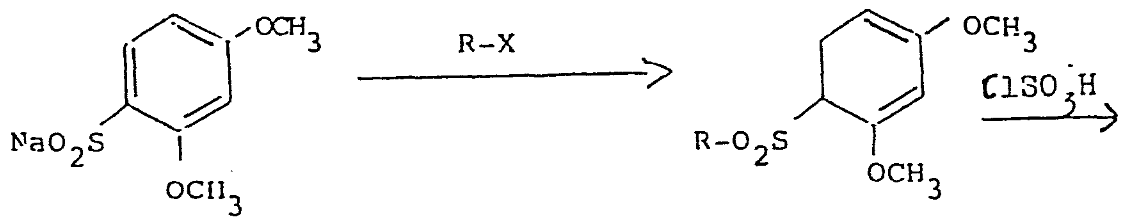


Diméthoxy-2,4 benzène-sulfinate de sodium

(Diméthoxy-2,4 phényl) alkylsulfone

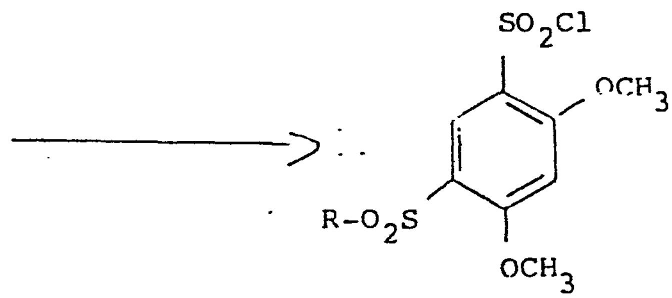

Chlorure d'alkylsulfonyl-5 diméthoxy-2,4 benzènesulfonyle

<u>Acide diméthoxy-2,4 benzènesulfinique</u> :

A une solution aqueuse contenant 129 g (0,974 mole) de sulfite de sodium sont ajoutés par petites portions et sous agitation 115 g (0,487 mole) de chlorure de diméthoxy-2,4 benzènesulfonyle. Durant cette opération, le pH est maintenu alcalin par addition de lessive de soude. Après 3 h de contact, le milieu réactionnel est filtré ; le filtrat est acidifié par de l'acide sulfurique 2N jusqu'à précipitation de l'acide sulfinique.

Rdt 72% F 122°C

<u>Diméthoxy-2,4 benzènesulfinate de sodium</u> :

Il est obtenu par addition de la quantité stoechiométrique d'hydroxyde de sodium en solution dans l'eau. La solution de sulfinate est évaporée à sec.

<u>(Diméthoxy-2,4 phényl) alkylsulfones</u> :

Protocole général :

Dans un ballon de 500 $cm^3$, muni d'un réfrigérant et d'un système d'agitation magnétique, sont ajoutés successivement 0,1 mole de diméthoxy-2,4 benzènesulfinate de sodium, 250 $cm^3$ d'isopropanol, puis 0,15 mole d'halogénure d'alkyle, de préférence un iodure. Le mélange est porté à l'ébullition à reflux 5 à 30 h selon l'halogénure mis en oeuvre. Après refroidissement, le milieu réactionnel est évaporé à sec. Le résidu est repris par de l'eau puis est extrait par du chloroforme. L'évaporation de la phase organique, après séchage sur sulfate de sodium fournit une huile qui est cristallisée dans du benzène.

Les produits suivants ont été obtenus selon cette méthode :

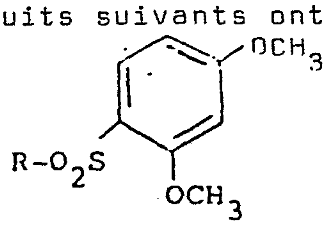

| R | PM | Temps de chauffage | F°C | Rdt % |
|---|---|---|---|---|
| $CH_3$ | 216 | 5 h | 109 | 65 |
| $C_2H_5$ | 230 | 7 h | 94 | 70 |
| $_nC_3H_7$ | 244 | 15 h | 70 | 90 |
| $_iC_3H_7$ | 244 | 30 h | 100 | 64 |

<u>Chlorures d'alkylsulfonyl-5 diméthoxy-2,4 benzènesulfonyles</u> :

Protocole général :

Dans un tricol d'un litre, muni d'un système d'agitation magnétique, d'une garde à chlorure de calcium, d'un thermomètre et d'une ampoule à réactif, est introduite une solution de (diméthoxy-2,4 phényl) alkylsulfone (0,2 mole) dans 200 $cm^3$ de chloroforme pur. Après refroidis-

BAD ORIGINAL

sement vers -10°C, sont ajoutés goutte à goutte 100 $cm^3$ d'acide chlorosulfonique. Une fois l'addition terminée, le mélange est laissé en contact 0,5 h à -10°C, puis est ramené à température ambiante et laissé sous agitation pendant 7 h; il est ensuite versé sur de la glace pilée. Le mélange obtenu est extrait par du chloroforme. La phase organique est séchée sur sulfate de sodium, filtrée, puis évaporée sous pression réduite. Le résidu est cristallisé dans du benzène.

Les produits suivants ont été obtenus selon cette méthode.

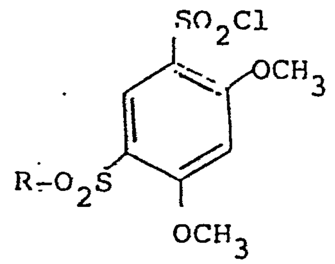

| R | PM | F°C | Rdt % |
|---|---|---|---|
| $CH_3$ | 314,5 | 204 | 52 |
| $C_2H_5$ | 328,5 | 191 | 77 |
| $_nC_3H_7$ | 342,5 | 169 | 87 |
| $_iC_3H_7$ | 342,5 | 218 | 66 |

Chlorure de diméthoxy-2,4 méthylsulfonyl-5 benzènesulfonyle

RMN (DMSO) à 80 MHz :

δ 8,2 ppm (s 1H ArH) ; δ 6,9 ppm (s 1H ArH) ; δ 4,1 ppm et 4,05 ppm (2s 6H $OCH_3$) ; 3,2 ppm (s 3H $CH_3$).

IR (KBr) :

ν $\underline{SO_2}$-Cl , as 1360 $cm^{-1}$ , s 1170 $cm^{-1}$ ;

ν $\underline{SO_2}$-$CH_3$, as 1300 $cm^{-1}$ , s 1140 $cm^{-1}$ .

Chlorure d'éthylsulfonyl-5 diméthoxy-2,4 benzènesulfonyle

RMN (DMSO) à 80 MHz :

δ 8,2 ppm (s 1H ArH) ; δ 6,93 ppm (s 1H ArH) ; δ 4,15 ppm et 4,1 ppm (2s 6H $OCH_3$) ; δ 3,25 ppm (q 2H $-CH_2-$) ; δ 1,18 ppm (t 3H $CH_3-$).

IR (KBr) :

ν $\underline{SO_2}$-Cl, as 1370 $cm^{-1}$, s 1175 $cm^{-1}$ ;

ν $\underline{SO_2}$-$CH_2$-, as 1315 $cm^{-1}$, s 1140 $cm^{-1}$.;

BAD ORIGINAL BAD ORIGINAL

Chlorure de diméthoxy-2,4 propylsulfonyl-5 benzènesulfonyle

RMN (DMSO) à 80 MHz :

δ 8,05 ppm (s 1H ArH) ; δ 6,72 ppm (s 1H ArH) ; 3,92 et 3,87 ppm (2s 6H $OCH_3$); δ 3,2 ppm (t 2H-$\underline{CH_2}$-$SO_2$-); δ 1,45 ppm (m 2H -$\underline{CH_2}$-); δ 0,85 ppm (t 3H $CH_3$).

IR (KBr) :

$\nu$ $\underline{SO_2}$Cl, as 1370 $cm^{-1}$, s 1180 $cm^{-1}$ ;

$\nu$ $\underline{SO_2}$-$CH_2$, as 1315 $cm^{-1}$, s 1135 $cm^{-1}$ .

Chlorure d'isopropylsulfonyl-5 diméthoxy-2,4 benzènesulfonyle

RMN (DMSO) à 80 MHz :

δ 8,02 ppm (s 1H ArH) ; δ 6,7 ppm (s 1H ArH);δ 3,92 et 3,85 ppm (2s 6H $OCH_3$) ; δ 3,48 ppm (m 1H -CH-) ; δ 1,11 et 1,03 ppm (2s 6H $CH_3$).

IR (KBr) :

$\nu$ $\underline{SO_2}$-Cl , as 1365 $cm^{-1}$, s 1175 $cm^{-1}$ ;

$\nu$ $\underline{SO_2}$-CH< , as 1300 $cm^{-1}$, s 1130 $cm^{-1}$ .

Préparation du chlorure de bromo-5 méthoxy-2 nitro-4 benzènesulfonyle

1ère étape : Obtention du bromo-4 nitro-2 anisole

A 0,7 mole (107, 1 g) d'ortho-nitroanisole en solution dans l'acide acétique (400 ml), on ajoute à chaud, goutte à goutte, 40 ml de brome. Une fois le dégagement d'acide bromhydrique terminé, on verse la solution dans de l'eau. On obtient un précipité que l'on dissout dans le chloroforme. La solution organique est séchée sur sulfate de sodium, puis évaporée.

Rdt : 66 %  F : 88°C

2ème étape : Obtention de l'amino-2 bromo-4 anisole

A 35 g de chlorure stanneux en solution dans 100 ml d'acide chlorhydrique (d : 1,18), on ajoute 0,05 mole (11,6 g) de bromo-4 nitro-2 anisole. Le mélange est porté deux heures à reflux. La solution est alcalinisée par de la soude, puis extraite par du chlorure de méthylène. La phase organique est séchée, puis évaporée.

Rdt : 60 %  F : 92°C

3ème étape : Obtention de l'acétamido-2 bromo-4 anisole

BAD ORIGINAL

A 0,3 mole (60 g) d'amino-2 bromo-4 anisole, on ajoute 100 ml d'éthanol, une pincée de poudre de zinc puis, par portions, 50 ml d'anhydride acétique.

La solution est portée une heure à reflux, puis versée sur de la glace pilée.

Rdt : 90 % F : 124°C

4ème étape : obtention de l'acétamido-2 bromo-4 nitro-5 anisole

A 0,2 mole (48 g) d'acétamido-2 bromo-4 anisole en solution dans 80 ml d'acide sulfurique de NORDHAUSEN, on ajoute en refroidissant 22 ml d'acide nitrique en solution dans 18 ml d'acide sulfurique de NORDHAUSEN. Après trois heures de contact, le mélange est versé sur de la glace pilée. Le précipité obtenu est extrait au dichlorométhane. La phase organique est séchée, puis évaporée. Les cristaux sont lavés à l'éther.

Rdt : 35 % F : 180°C

5ème étape : obtention de l'amino-2 bromo-4 nitro-5 anisole

A 9,5 g d'acétamido-2 bromo-4 nitro-5 anisole on ajoute 8,8 g de potasse dans 40 ml d'eau. Le mélange est porté deux heures à reflux, puis versé sur de la glace pilée. Le résidu est cristallisé dans de l'éthanol.

Rdt : 60 % F : 130°C

6ème étape : obtention du chlorure de bromo-5 méthoxy-2 nitro-4 benzènesulfonyle

0,1 mole (24,7 g) d'amino-2 bromo-4 nitro-5 anisole est diazotée par du nitrite de sodium en milieu chlorhydrique.

Le sel de diazonium est versé dans une solution saturée d'anhydride sulfureux dans l'acide acétique (150 ml) en présence de chlorure cuivrique (3 g).

Après la fin du dégagement d'azote, le mélange est versé sur de la glace pilée. Le résidu est cristallisé dans un mélange dichlorométhane/éther de pétrole. Rdt : 40 % F : 127°C

Ce composé est un composé nouveau.

Préparation de l'aminométhyl-2 éthyl-1 azépine

1ère étape : préparation de l'éthyl-1 caprolactame

A 5 g (0,2 mole) d'hydrure de sodium en suspension dans 250 ml de xylène, est ajoutée goutte à goutte une solution de 22,6 g (0,2 mole) de caprolactame dans 150 ml de xylène.

Après un reflux d'une heure, 31,2 g (0,2 mole) d'iodure d'éthyle sont ajoutés à 5°C au mélange réactionnel.

Après deux heures au reflux, filtration et évaporation à sec sous vide, l'huile obtenue est distillée.

Rdt : 80 %    Eb (2 mmHg) : 72°C

2ème étape : préparation de l'éthyl-1 nitrométhylène-2 azépine

A 14,1 g (0,1 mole) d'éthyl-1 caprolactame sont ajoutés 12,6 g (0,1 mole) de sulfate de méthyle. Le milieu réactionnel est porté pendant 90 minutes à 65°C. Après refroidissement, 30 ml d'une solution 3,25 N de méthylate de sodium dans le méthanol sont ajoutés en trente minutes, puis 0,15 g (0,15 ml) de nitrométhane.

Après une nuit d'agitation, le milieu réactionnel évaporé à sec sous vide est repris dans 20 ml d'eau. Le précipité obtenu en quantité de 9,5 g est essoré puis séché sous vide.    Rdt : 52 %    F : 121°C

3ème étape : préparation de l'aminométhyl-2 éthyl-1 azépine

A 4 g (0,022 mole) de l'éthyl-1 nitrométhylène-2 azépine dans 100 ml de tétrahydrofuranne anhydre sont ajoutés 3,5 g d'hydrure double de lithium et d'aluminium. Après cinq heures à reflux, hydrolyse et évaporation de la phase organique, l'amine (2,9 g) est obtenue sous forme d'une huile incolore.

Rdt : 75 %

Préparation de l'aminométhyl-2 benzyl-1 pyrrolidine

On opère suivant la technique décrite par H. REITSEMA et reportée au J. Of American Chemical Soc. (1949), p. 2 041-2 042.

Le chlorhydrate de la N-benzyl chloro-3 pipéridine, à raison de 49,4 g (0,2 mole) est ajouté à 50 cm3 d'eau additionnée de 42,8 g (0,4 mole) de benzylamine.

Le mélange est porté à une température de 65 à 75°C. Après 48 heures, le milieu réactionnel est additionné de 60 cm3 d'eau, puis alcalinisé par une quantité suffisante de carbonate de potassium pour établir le pH à environ 10. On extrait par de l'éther éthylique. La phase organique récupérée est séchée sur sulfate de sodium et on évapore le solvant. Le résidu est distillé sous vide poussé (0,01 mmHg) à 110°C. On obtient ainsi par distillation le benzyl-1 benzylamino-méthyl-2 pyrrolidine.

Le produit obtenu précédemment est soumis à une hydrogénolyse catalytique en présence de charbon palladié à 10 %, dans 100 cm3 d'éthanol absolu et à 50°C. Lorsque la quantité théorique d'hydrogène nécessaire pour l'hydrogénolyse complète a été absorbée, on arrête la réaction.

Après filtration du catalyseur et lavage de celui-ci, le filtrat est distillé sous pression réduite à une température inférieure à 45°C. On obtient par distillation l'aminométhyl-2 benzyl-1 pyrrolidine avec un rendement de 97 %.

De façon similaire, a été préparée : l'aminométhyl-2 cyclopropylméthyl-1 pyrrolidine.

Les exemples suivants sont relatifs à la préparation de certains composés racémiques selon l'invention.

EXEMPLE 1 : préparation du chloro-5 diméthoxy-2,4 N(N-benzyl-méthyl-2 pyrrolidino)benzènesulfonamide

On condense 27,1 g de chlorure de chloro-5 diméthoxy-2,4 benzène sulfonyle et 12,8 g d'aminométhyl-2 benzyl-1 pyrrolidine de la façon suivante : on dissout le sulfochlorure dans 150 ml de benzène anhydre, on ajoute goutte à goutte sous agitation l'aminométhyl-2

benzyl-1 pyrrolidine ; la solution s'échauffe, puis se trouble. Le chlorhydrate obtenu précipite ; on le sépare pour le cristalliser dans de l'isopropanol ; rendement : 95 %.

EXEMPLES 2 A 14

Un certain nombre d'autres racémiques conformes à l'invention ont été obtenus en mettant en oeuvre les chlorures de benzènesulfonyle et les amines racémiques appropriées et sont rassemblés dans le tableau suivant.

TABLEAU A

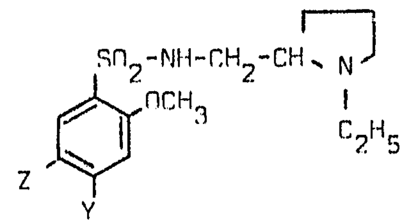

| Composé n° | Y | Z | Formule brute, HCl | Poids moléculaire | Point de fusion °C | Rendement % |
|---|---|---|---|---|---|---|
| | $NO_2$ | H | $C_{14}H_{22}ClN_3O_5S$ | 379,87 | 140 | 80 |
| 101 | H | Cl | $C_{14}H_{22}Cl_2N_2O_3S$ | 369,30 | 168 | 70 |
| 204 | H | $NO_2$ | $C_{14}H_{22}ClN_3O_5S$ | 379,87 | 218 * | 75 |
| 640 | H | Br | $C_{14}H_{22}BrClN_2O_3S$ | 413,75 | 156 * | 80 |
| | $NO_2$ | Br | $C_{14}H_{21}BrClN_3O_5S$ | 458,75 | 242 | 75 |
| 885 | $OCH_3$ | Cl | $C_{15}H_{24}Cl_2N_2O_4S$ | 399,33 | 198 | 95 |
| 892 | $OCH_3$ | $OCH_3$ | $C_{16}H_{27}ClN_2O_5S$ | 394,91 | 158 | 50 |
| 929 | $OCH_3$ | Br | $C_{15}H_{24}BrClN_2O_4S$ | 443,78 | 213 | 42 |
| 960 | $OCH_3$ | $-SO_2CH_3$ | $C_{16}H_{27}ClN_2O_6S_2$ | 442,96 | 206 | 71 |
| 984 | $OCH_3$ | $-SO_2C_2H_5$ | $C_{17}H_{29}ClN_2O_6S_2$ | 456,99 | 228 | 85 |
| 1 066 | $OCH_3$ | H | $C_{15}H_{25}ClN_2O_4S$ | 364,88 | 145<br>91 * | 77 |
| 1 099 | $OCH_3$ | $-SO_{2n}C_3H_7$ | $C_{18}H_{31}ClN_2O_6S_2$ | 471,02 | 189 | 80 |
| 1 109 | $OCH_3$ | $-SO_{2i}C_3H_7$ | $C_{18}H_{31}ClN_2O_6S_2$ | 471,02 | 202 | 79 |

* Le point de fusion est celui de la base

EXEMPLE 15 : préparation du chloro-5 méthoxy-2 nitro-4 N-(N-éthyl méthyl-2 azépinyl) benzènesulfonamide

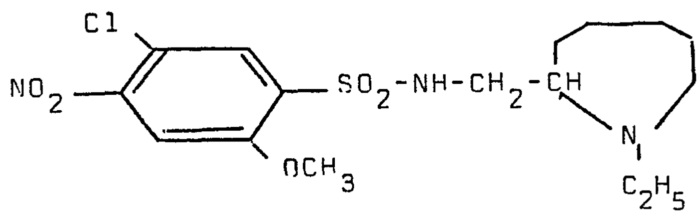

A 5,92 g (0,02 mole) de chlorure de chloro-5 méthoxy-2 nitro-4 benzènesulfonyl dans 100 ml de chlorure de méthylène à 0°C, 3,2 g (0,02 mole) d'aminométhyl-2 éthyl-1 azépine sont ajoutés goutte à goutte. Après une nuit sous agitation le mélange est évaporé à sec puis repris dans l'eau. Après alcalinisation à pH 10 par de la soude 2N, le produit est extrait à l'acétate d'éthyle. Après évaporation à sec le produit est recristallisé dans l'éther isopropylique.

EXEMPLE 16 : préparation de l'amino-4 chloro-5 méthoxy-2 N-(N-éthyl méthyl-2 azépinyl) benzènesulfonamide (composé n° 1 149)

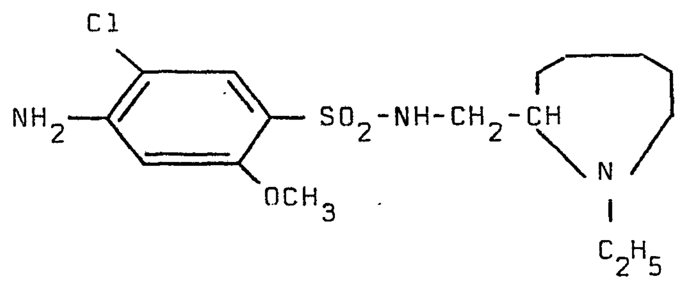

A 2 g (0,005 mole) du composé nitré de l'exemple précédent dans 100 ml de méthanol anhydre, 200 mg de platine Adams sont ajoutés. Après absorption de la quantité théorique $H_2$, le catalyseur est éliminé. Après pressage à la base le produit est cristallisé sous forme de picrate. F : 174°C.

EXEMPLES 17 ET 18

En partant des aminométhyl-2 azépines obtenues selon le procédé décrit précédemment, et par

condensation avec les chlorures de benzènesulfonyle, on obtient les produits suivants :

- le chloro-5 diméthoxy-2,4 N-(N-éthyl méthyl-2 azépinyl) benzènesulfonamide de formule :

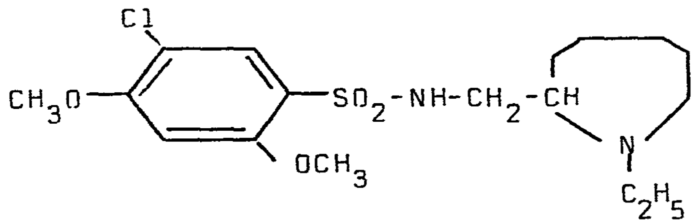

- le triméthoxy-2,4,5 N-(N-éthyl méthyl-2 azépinyl) benzènesulfonamide de formule :

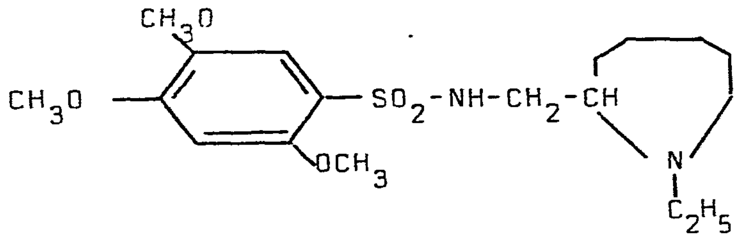

EXEMPLES 19 A 23

En condensant mole par mole un sulfochlorure avec une aminométhyl 3 ou 4 benzyl 1 (substitué ou non) pipéridine, on obtient des pipéridino 3 et 4 benzènesulfonamides.

Les pipéridines utilisées sont les suivantes :

- l'aminométhyl-4 benzyl-1 pipéridine,
- l'aminométhyl-4 (chloro-4 benzyl)-1 pipéridine,
- l'aminométhyl-3 benzyl-1 pipéridine,
- l'aminométhyl-3 (chloro-4 benzyl)-1 pipéridine.

Les sulfochlorures utilisés sont les suivants :

- chlorure de chloro-5 diméthoxy-2,4 benzènesulfonyle,
- chlorure de chloro-5 méthoxy-2 nitro-4 benzènesulfonyle.

On obtient les produits suivants :

- N-/¯(benzyl-1 pipéridinyl-4)méthyl_7 chloro-5 diméthoxy-2,4 benzènesulfonamide ;
- chloro-5 N-/ /¯chloro-4 benzyl)-1 pipéridinyl-4_7 méthyl_/diméthoxy-2,4 benzènesulfonamide ;
- N-/¯(benzyl-1 pipéridinyl-3)méthyl_7chloro-5 diméthoxy-2,4 benzènesulfonamide ;

- amino-4 N-/¯(benzyl-1 pipéridinyl-3)méthyl_7chloro-5 méthoxy-2 benzènesulfonamide ;
- amino-4 N-/¯(benzyl-1 pipéridinyl-4)méthyl_7chloro-5 méthoxy-2 benzènesulfonamide.

SYNTHESE DES ISOMERES OPTIQUES

Les exemples qui suivent relatifs à l'obtention d'isomères optiques ont été obtenus par dédoublement du racémique correspondant.

EXEMPLE 24 : préparation des isomères optiques du N-/¯(benzyl-1 pyrrolidinyl-2)méthyl_7chloro-5 diméthoxy-2,4 benzènesulfonamide

Ils sont obtenus par salification de l'amine par la quantité stoechiométrique d'acide tartrique optiquement actif. Les sels obtenus sont cristallisés plusieurs fois dans les alcools, de préférence méthanol ou éthanol, jusqu'à l'obtention d'une pureté optique supérieure à 95 %. Par alcalinisation, les bases sont libérées, recristallisées et présentent les caractéristiques suivantes :

Isomère lévogyre :

(Isomère S) : F = 112°C $\{\alpha\}_D^{20}$ = - 22,8°
c = 1 % dans le méthanol
$\{\alpha\}_{365}^{20}$ = - 74,1°
c = 1 % dans le méthanol

Isomère dextrogyre :

(Isomère R) : F = 110/111°C $\{\alpha\}_D^{20}$ = + 22,5°
c = 1 % dans le méthanol
$\{\alpha\}_{365}^{20}$ = + 75,0°
c = 1 % dans le méthanol

EXEMPLES 25 A 31

Les isomères optiques du tableau B suivant ont été obtenus par dédoublement des racémiques des pyrrolidinobenzènesulfonamides correspondants.

Les isomères optiques de ce tableau B peuvent également être obtenus à partir des isomères optiques des amines obtenues par dédoublement des racémiques par l'acide tartrique, ces amines dissoutes étant ensuite

condensées avec les sulfochlorures appropriés.

On peut également obtenir les isomères optiques actifs correspondant aux racémiques du tableau A de façon similaire.

TABLEAU B

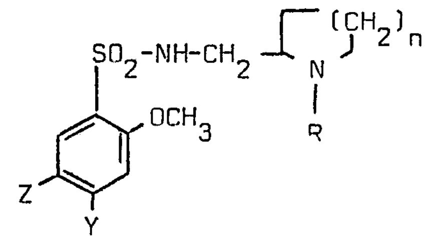

| Composé n° | Z | Y | R | n | Formule | Masse moléculaire | F°C | Pouvoir rotatoire |
|---|---|---|---|---|---|---|---|---|
| 1 135 (°) | Cl | $NH_2$ | $C_2H_5$ | 1 | $C_{14}H_{22}ClN_3O_3S$ isomère S | 347,86 | | $\{\alpha\}_D^{20}$=-46,0° c=0,5%$CH_3OH$ $\{\alpha\}_{436}^{20}$=-94,2° c=0,5%$CH_3OH$ |
| 1 139 | $OCH_3$ | $OCH_3$ | $C_2H_5$ | 1 | $C_{16}H_{26}N_2O_5S$, HCl isomère S | 394,93 | 128 | $\{\alpha\}_D^{20}$=+13,5°c=1% $CH_3OH$ $\{\alpha\}_{365}^{20}$=+87,9° c=1% $CH_3OH$ |
| 1 139 | $OCH_3$ | $OCH_3$ | $C_2H_5$ | 1 | $C_{16}H_{26}N_2O_5S$ isomère S | 358,47 | 115 | $\{\alpha\}_D^{20}$=-49,0° c=1% $CH_3OH$ $\{\alpha\}_{365}^{20}$=-177,8° c=1%$CH_3OH$ |
| 1 138 | Cl | $OCH_3$ | $C_2H_5$ | 1 | $C_{15}H_{23}ClN_2O_4S$ HCl isomère S | 399,35 | 215 | $\{\alpha\}_D^{20}$=+14,5° c=1% $CH_3OH$ $\{\alpha\}_{365}^{20}$=+82,1° c=1% $CH_3OH$ |

Suite TABLEAU B

| Composé n° | Z | Y | R | n | Formule | Masse moléculaire | F°C | Pouvoir rotatoire |
|---|---|---|---|---|---|---|---|---|
| 1 138 | Cl | $OCH_3$ | $C_2H_5$ | 1 | $C_{15}H_{23}ClN_2O_4S$ isomère S | 362,89 | 125 | $\{\alpha\}_D^{20}$=-46,1° c=1% $CH_3OH$ <br> $\{\alpha\}_{365}^{20}$=-163,1° c=1% $CH_3OH$ |
| 1 137 | 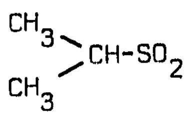 | $OCH_3$ | $C_2H_5$ | 1 | $C_{12}H_{30}N_2C_6S_2$ isomère S | 434,59 | 181 | $\{\alpha\}_D^{20}$=-38,8° c=1% $CH_3OH$ <br> $\{\alpha\}_{365}^{20}$=-134,8° c=1% $CH_3OH$ |
| 1 136 | Cl | $OCH_3$ | $CH_2$- 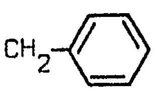 | 1 | $C_{20}H_{25}ClN_2O_4S$ isomère S | 424,96 | 112 | $\{\alpha\}_D^{20}$ =-22,8° c=1% $CH_3OH$ <br> $\{\alpha\}_{365}^{20}$=-74,1° c=1% $CH_3OH$ |
| 1 179 | Cl | $OCH_3$ | $CH_2$- 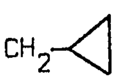 | 1 | $C_{17}H_{25}ClN_2O_4$ picrate isomère S | 618,04 | 172 | $\{\alpha\}_{365}^{20}$=-2,2° c=0,4%$CH_3OH$ |
| 1 134 | Cl | $NO_2$ | $C_2H_5$ | 1 | $C_{14}H_{20}ClN_3O_5S$ HCl | 414,33 | 210 | $\{\alpha\}_D^{20}$=+2,2° c=0,5%$CH_3OH$ <br> $\{\alpha\}_{436}^{20}$ =+19,8° c=0,5%$CH_3OH$ |

(°) note : en ce qui concerne le composé n° 1 135, il s'agit de l'isomère optique lévogyre sous forme de base du composé racémique décrit dans le brevet français n° 2 386 524.

Les nouveaux benzènesulfonamides selon l'invention, ainsi que leurs sels organiques ou minéraux physiologiquement acceptables présentent de remarquables propriétés pharmacologiques.

Les composés selon l'invention exercent une action sur le système nerveux central et sont utilisés avantageusement à titre de principes actifs dans le traitement des affections gastro-intestinales d'origine psychosomatique.

Les composés selon l'invention sont dépourvus de toxicité.

Plus particulièrement, les composés selon l'invention ainsi que leurs sels organiques ou minéraux présentent notamment soit des propriétés anti-ulcéreuses, soit des propriétés anti-émétiques.

Les composés présentant d'intéressantes propriétés anti-ulcéreuses sont les racémiques, en particulier ceux de formule (VII).

Ces composés sont par conséquent avantageusement introduits comme principes actifs dans les médicaments pour le traitement des maladies ulcéreuses gastroduodénales.

Les composés présentant d'intéressantes propriétés anti-émétiques sont principalement les composés lévogyres, en particulier ceux rassemblés dans le tableau B, et notamment les composés n° 1 135, 1 138, ainsi que les racémiques du tableau A, en particulier le composé n° 1 149, et leurs sels organiques ou minéraux physiologiquement acceptables.

Ils sont avantageusement introduits comme principes actifs dans le traitement des maladies gastroduodénales.

Dans ces médicaments selon l'invention, les substances actives sont associées, dans la mesure où cela est nécessaire, avec des excipients et adjuvants traditionnels destinés à faciliter et à améliorer leur utilisation, leur conservation, etc.

Compte tenu de leurs activités et des traitements pour lesquels ils sont utilisés, les médicaments selon l'invention peuvent être administrés par voie parentérale. A cet effet, ils sont présentés sous forme de solutions stériles ou stérilisables, injectables ou propres à être utilisées, pour la préparation extemporanée de solutions injectables.

Ces exemples ne présentant bien entendu aucun caractère limitatif quant à la définition des produits physiologiquement acceptables peuvent être utilisés pour former des solutions injectables.

Les médicaments selon l'invention peuvent également être administrables par d'autres voies et notamment par voie orale. Administrés par voie orale, ils sont présentés sous des formes très variées : capsules, gélules, solutions, suspensions, sirops; par voie topique, ils sont présentéssous forme de crème, pommade, lotion, gel, etc.

Les doses administrées peuvent varier selon le mode d'administration et la phase de traitement.

Dans des présentations pharmaceutiques pour administration par voie parentérale, la dose de produit par unité est comprise d'environ 10 à environ 200 mg.

Par voie orale, la dose unitaire est comprise d'environ 10 à environ 500, de préférence d'environ 50 à environ 250 mg.

Par voie topique, les présentations comportent de 1 à 20 % en poids de la présentation. Les doses journalières à administrer par kg de poids du malade sont de l'ordre de 0,1 à 5 mg par voie injectable et de l'ordre de 1 à 10 mg par voie orale.

Les résultats des tests indiqués ci-après montrent les propriétés anti-émétiques des composés selon l'invention.

On donne les résultats pour les composés suivants :

- le composé racémique n° 1 149 (sous forme

de base) ;

- l'isomère optique lévogyre n° 1 138 ;
- l'isomère optique lévogyre n° 1 135 ;
- le racémique du lévogyre n° 1 135.

Les tests ont été réalisés comme suit.

L'étude a été effectuée sur des chiens Beagle provenant de l'élevage du Centre de Recherches Biologiques de Baugy.

On a tout d'abord effectué un essai témoin, puis six jours après, on a testé notamment les quatre composés mentionnés ci-dessus.

Pendant l'essai témoin, les animaux reçoivent 100 µg/kg d'apomorphine, qui provoque des vomissements.

Lorsqu'on teste les produits conformes à l'invention et le racémique du composé n° 1 135, les animaux reçoivent :

- à l'instant 0, une injection intra-musculaire du composé étudié à raison d'environ 1 mg/kg ;
- 30 minutes plus tard, une injection sous-cutanée d'apomorphine est effectuée, d'environ 100 µg/kg.

Les résultats donnés ci-après correspondent aux pourcentages de diminution du nombre de vomissements par rapport à l'essai témoin.

| Produit n° | Diminution par rapport au témoin (%) |
|---|---|
| 1 149 | 60 |
| 1 135 | 50 |
| 1 138 | 20 |
| racémique du lévogyre n° 1 135 | 10 |

On constate que tous les produits provoquent une diminution du nombre de vomissements et que l'isomère lévogyre n° 1 135 est beaucoup plus actif que le racémique correspondant, la différence d'activité qui résulte de la mise en oeuvre comparative de ces deux composés montre qu'il s'agit véritablement d'une différence de nature.

REVENDICATIONS

1. Composé lévogyre répondant à la formule générale (I) suivante :

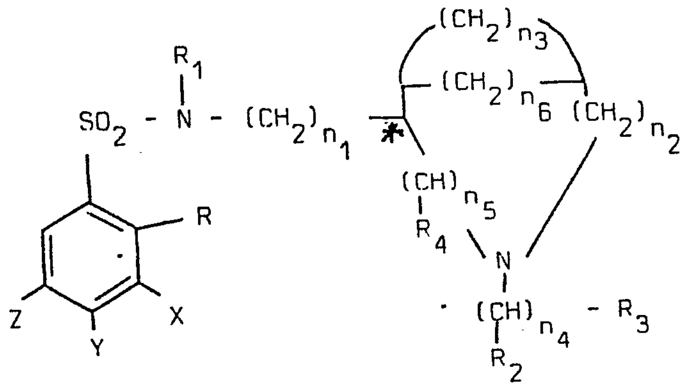

(I)

dans laquelle :

- $n_1$ varie de 0 à 3,
- $n_2$ peut varier de 1 à 4, et peut prendre la valeur 0, lorsque $n_5$ est différent de 0,
- $n_3$ prend les valeurs 0, 3, 4, 5 ou 6,
- $n_4$ varie de 0 à 4,
- $n_5$ varie de 0 à 2,
- $n_6$ varie de 0 à 2,
- R représente un radical alcoxy de 1 à 6 atomes de carbone, de préférence méthoxy ou éthoxy, ou un radical alcényloxy de 2 à 6 atomes de carbone, de préférence de 3 atomes de carbone ;
- $R_1$ représente un atome d'hydrogène, un radical alcoyle linéaire ou ramifié de 1 à 6 atomes de carbone, un radical cycloalcoyle de 3 à 6 atomes de carbone, un radical alcényle de 2 à 6 atomes de carbone, de préférence de 3 à 6 atomes de carbone, un radical cycloalcényle de 3 à 6 atomes de carbone, de préférence un radical cyclopentène ou cyclohexène, un radical benzyle substitué ou non :

  - par un atome d'halogène, de préférence le fluor,

  - par un radical alcoxy inférieur de 1 à 4 atomes de carbone,

  - par un radical alcoyle linéaire ou ramifié de 1 à 4 atomes de carbone ;

- $R_2$ et $R_3$ représentent, indépendamment l'un de l'autre, un atome d'hydrogène, un radical alcényle de 2 à 6 atomes de carbone, de préférence de 3 à 6 atomes de carbone, un radical cycloalcényle de 3 à 6 atomes de carbone, de préférence un radical cyclopentène ou cyclohexène, un radical phényle substitué ou non :
  - par un atome d'halogène, de préférence le fluor,
  - par un radical alcoxy inférieur de 1 à 4 atomes de carbone,
  - par un radical alcoyle linéaire ou ramifié de 1 à 4 atomes de carbone ;
- $R_4$ représente un atome d'hydrogène, un radical alcoyle de 1 à 6 atomes de carbone ou un radical alcényle de 2 à 6 atomes de carbone ;
- X représente un atome d'hydrogène, un halogène, le groupe $OCH_3$, $CF_3$, $NO_2$ ou $NH_2$ ;
- Y représente un atome d'hydrogène, un halogène, un radical acylamino de 2 à 4 atomes de carbone, le groupe $OCH_3$, $CF_3$, $NO_2$ ou $NH_2$ ;
- Z représente un hydrogène, un halogène, le groupe $OCH_3$, $NO_2$, $NH_2$, $CF_3$, CN, $-\underset{\underset{O}{\|}}{C}-R_5$, $R_5$ étant un radical alcoyle de 1 à 6 atomes de carbone, notamment éthyle ou méthyle, $-SR_6$, $R_6$ représentant un radical alcoyle linéaire ou ramifié ayant de 1 à 4 atomes de carbone, notamment un radical méthyle ou éthyle, $SO_2CH_3$, $SO_2C_2H_5$, $SO_{2n}C_3H_7$, $SO_{2i}C_3H_7$, $SO_2N\begin{matrix}-R_7\\ \diagdown R_8\end{matrix}$, $R_7$ et $R_8$ représentant, indépendamment l'un de l'autre, un atome d'hydrogène, un radical alcoyle de 1 à 6 atomes de carbone, et de préférence un hydrogène ou un méthyle, ainsi que leurs sels obtenus avec des acides organiques ou minéraux physiologiquement acceptables.

2. Composé lévogyre selon la revendication 1 répondant à la formule générale suivante :

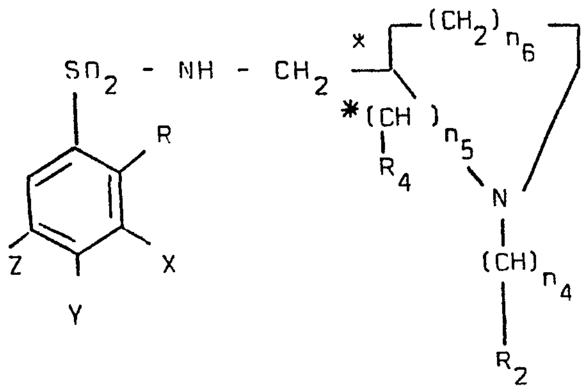

(III)

dans laquelle $n_4$, $n_5$, $n_6$, R, $R_2$, $R_3$, $R_4$, $R_5$, X, Y et Z ont les significations indiquées à la revendication 1.

3. Composé lévogyre selon la revendication 1 ou 2, répondant à la formule générale suivante :

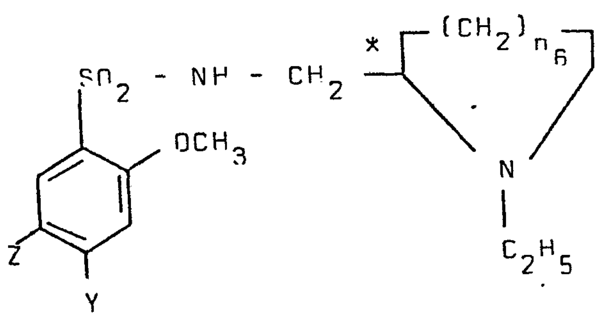

(VI)

dans laquelle :

- $n_6$ varie de 0 à 2,
- Y est choisi dans le groupe constitué par H, $NH_2$, $NO_2$, $OCH_3$,
- Z est choisi dans le groupe constitué par H, Cl, Br, $NO_2$, $OCH_3$, $SO_2CH_3$, $SO_2C_2H_5$, $SO_{2n}C_3H_7$, $SO_{2i}C_3H_7$.

4. Composé lévogyre selon la revendication 1 ou 2, répondant à la formule suivante :

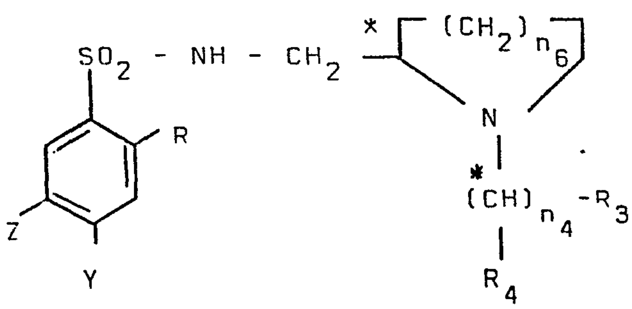

(X)

dans laquelle Y et Z ont les significations indiquées dans la revendication 1, R représente un groupe alcoxy de 1 à 6 atomes de carbone, notamment méthoxy ou éthoxy,

$n_6$ peut varier de 0 à 2 et $(\underset{|\atop R_4}{CH})_{n_4}-R_3$ représente l'un des radicaux suivants :

$C_2H_5$, 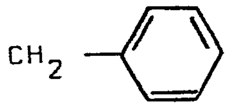 ou 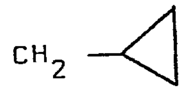

5. Composé lévogyre selon la revendication 4 répondant à la formule suivante :

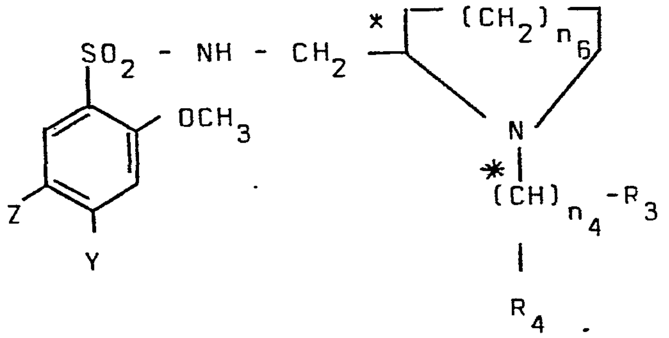

(XI)

dans laquelle $n_6$, $(\underset{|\atop R_4}{CH})_{n_4}-R_3$ ont les significations indiquées à la revendication 4, Y représente $OCH_3$, $NO_2$, $NH_2$ et Z représente Cl, $OCH_3$, $(CH_3)_2CH-SO_2$.

6. Composé lévogyre selon la revendication 5, répondant à la formule suivante :

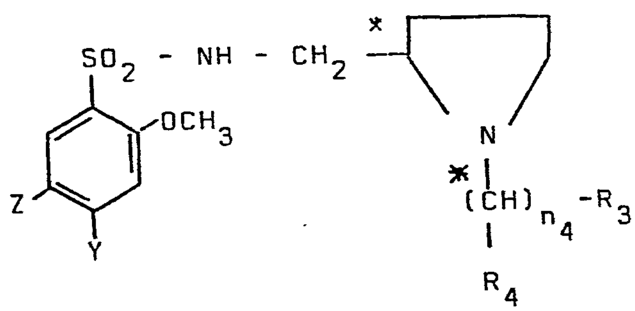

(XII)

dans laquelle Y, Z et $(\underset{|\atop R_4}{CH})_{n_4}-R_3$ ont les significations indiquées à la revendication 5.

7. Composés lévogyres de formules suivantes :

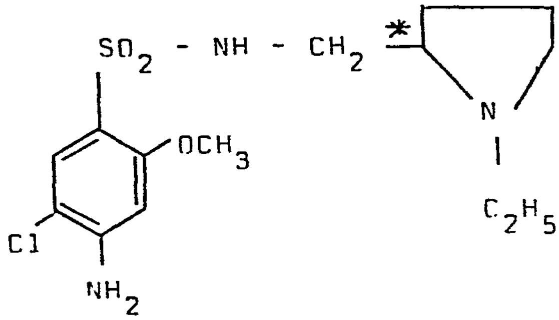

, HCl

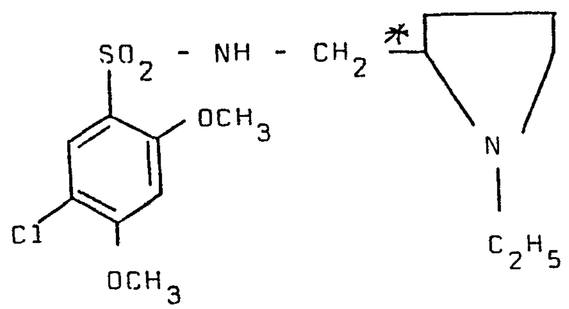

8. Procédé de préparation du chlorhydrate des composés lévogyres selon l'une quelconque des revendications 1 à 7, caractérisé en ce que :

- soit on prépare le composé racémique en faisant réagir le racémique d'un composé de formule :

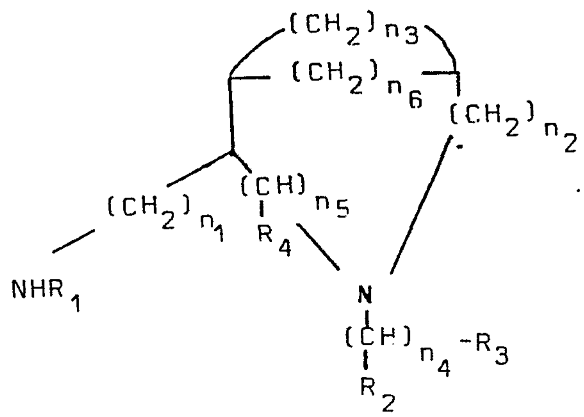

(XXIII)

dans laquelle $R_1$, $R_2$, $R_3$, $R_4$, $n_1$, $n_2$, $n_3$, $n_4$, $n_5$ et $n_6$ ont les significations indiquées aux revendications 1 à 7, sur un sulfochlorure de formule :

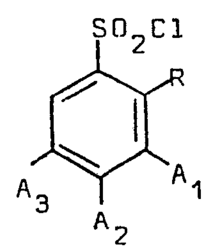

(XV)

dans laquelle R a la signification indiquée aux revendications 1 à 7, et $A_1$, $A_2$, $A_3$ ont les significations respectives de X, Y, Z à l'exception de $NH_2$, suivi d'une éventuelle réduction lorsque l'un au moins des X, Y, Z représente le groupe $NO_2$ ;

- puis on dédouble le racémique obtenu par addition d'acide tartrique optiquement actif, puis alcalinisation ;

- soit on fait réagir directement l'isomère optique de l'amine de formule :

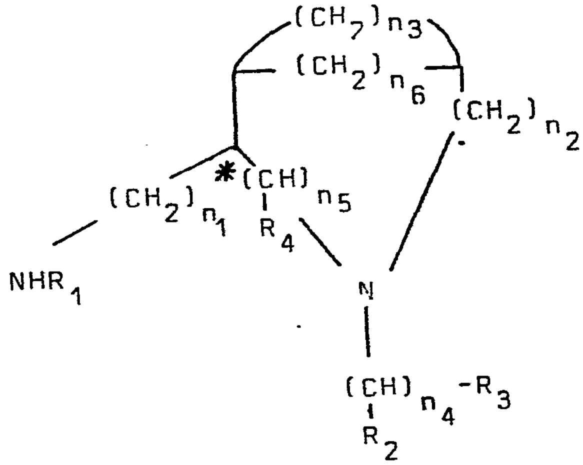

(XXIII)

cet isomère ayant été obtenu par dédoublement à l'acide tartrique du racémique de l'amine correspondante, sur le sulfochlorure de formule :

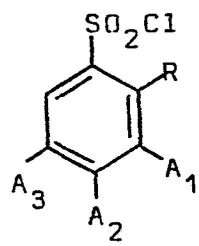

(XV)

dans laquelle R a la signification indiquée aux revendications 1 à 7 et $A_1$, $A_2$ et $A_3$ ont les significations ci-dessus indiquées.

9. Procédé de préparation des composés ou de leurs sels organiques ou minéraux physiologiquement acceptables, selon l'une quelconque des revendications 1 à 7, caractérisé en ce que le produit obtenu sous forme de chlorhydrate selon la revendication 8 est transformé en la base correspondante ou bien en un sel différent suivi éventuellement d'une réduction lorsque l'un des $A_1$, $A_2$, $A_3$ représente le groupe $NO_2$.

10. Procédé de préparation selon l'une quelconque des revendications 8 ou 9, caractérisé en ce que

l'amine est choisie parmi :

- l'aminométhyl-2 éthyl-1 pyrrolidine,
- l'aminométhyl-2 benzyl-1 pyrrolidine,
- l'aminométhyl-2 cyclopropyl-1 pyrrolidine,
- l'aminométhyl-2 éthyl-1 azépine,
- l'aminométhyl-3 benzyl-1 pipéridine,
- l'aminométhyl-3 (chloro-4 benzyl)-1 pipéridine,
- l'aminométhyl-4 benzyl-1 pipéridine,
- l'aminométhyl-4 (chloro-4 benzyl)-1 pipéridine.

11. Procédé de préparation selon l'une quelconque des revendications 8 à 10, caractérisé en ce que le sulfochlorure de formule (XV) est obtenu à partir des arylamines suivantes :

- diméthoxy-2,4 aniline,
- chloro-5 diméthoxy-2,4 aniline,
- bromo-5 diméthoxy-2,4 aniline,
- triméthoxy-2,4,5 aniline,
- diméthoxy-2,4 méthylsulfonyl-5 aniline,
- diméthoxy-2,4 éthylsulfonyl-5 aniline,
- diméthoxy-2,4 propylsulfonyl-5 aniline.
- diméthoxy-2,4 isopropylsulfonyl-5 aniline.

12. Composition pharmaceutique caractérisée en ce qu'elle contient l'un des composés ou leurs sels organiques ou minéraux physiologiquement acceptables selon l'une quelconque des revendications 1 à 7.

0088849

REVENDICATIONS AT

1. Procédé de préparation du chlorhydrate des composés lévogyres de formule générale (I) :

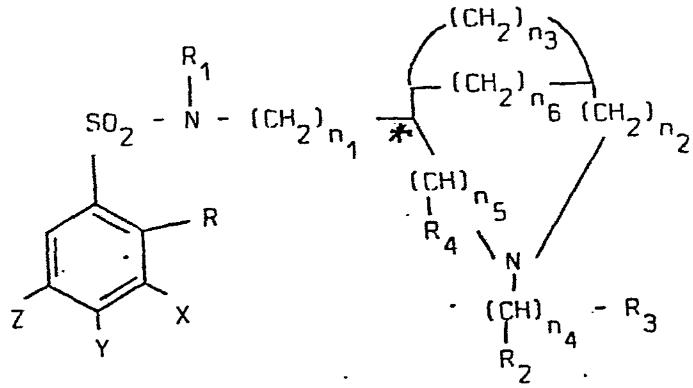

(I)

dans laquelle :

- $n_1$ varie de 0 à 3,
- $n_2$ peut varier de 1 à 4, et peut prendre la valeur 0, lorsque $n_5$ est différent de 0,
- $n_3$ prend les valeurs 0, 3, 4, 5 ou 6,
- $n_4$ varie de 0 à 4,
- $n_5$ varie de 0 à 2,
- $n_6$ varie de 0 à 2,
- R représente un radical alcoxy de 1 à 6 atomes de carbone, de préférence méthoxy ou éthoxy, ou un radical alcényloxy de 2 à 6 atomes de carbone, de préférence de 3 atomes de carbone ;
- $R_1$ représente un atome d'hydrogène, un radical alcoyle linéaire ou ramifié de 1 à 6 atomes de carbone, un radical cycloalcoyle de 3 à 6 atomes de carbone, un radical alcényle de 2 à 6 atomes de carbone; de préférence de 3 à 6 atomes de carbone, un radical cycloalcényle de 3 à 6 atomes de carbone, de préférence un radical cyclopentène ou cyclohexène, un radical benzyle substitué ou non :

  - par un atome d'halogène, de préférence le fluor,
  - par un radical alcoxy inférieur de 1 à 4 atomes de carbone,
  - par un radical alcoyle linéaire ou ramifié de 1 à 4 atomes de carbone ;

- $R_2$ et $R_3$ représentent, indépendamment l'un de l'autre, un atome d'hydrogène, un radical alcényle de 2 à 6 atomes de carbone, de préférence de 3 à 6 atomes de carbone, un radical cycloalcényle de 3 à 6 atomes de carbone, de préférence un radical cyclopentène ou cyclohexène, un radical phényle substitué ou non :

  - par un atome d'halogène, de préférence le fluor,

  - par un radical alcoxy inférieur de 1 à 4 atomes de carbone,

  - par un radical alcoyle linéaire ou ramifié de 1 à 4 atomes de carbone ;

- $R_4$ représente un atome d'hydrogène, un radical alcoyle de 1 à 6 atomes de carbone ou un radical alcényle de 2 à 6 atomes de carbone ;
- X représente un atome d'hydrogène, un halogène, le groupe $OCH_3$, $CF_3$, $NO_2$ ou $NH_2$ ;
- Y représente un atome d'hydrogène, un halogène, un radical acylamino de 2 à 4 atomes de carbone, le groupe $OCH_3$, $CF_3$, $NO_2$ ou $NH_2$ ;
- Z représente un hydrogène, un halogène, le groupe $OCH_3$, $NO_2$, $NH_2$, $CF_3$, CN, $-\underset{O}{\underset{\|}{C}}-R_5$, $R_5$ étant un radical alcoyle de 1 à 6 atomes de carbone, notamment éthyle ou méthyle, $-SR_6$, $R_6$ représentant un radical alcoyle linéaire ou ramifié ayant de 1 à 4 atomes de carbone, notamment un radical méthyle ou éthyle, $SO_2CH_3$, $SO_2C_2H_5$, $SO_{2n}C_3H_7$, $SO_{2i}C_3H_7$, $SO_2N{<}^{R_7}_{R_8}$, $R_7$ et $R_8$ représentant, indépendamment l'un de l'autre, un atome d'hydrogène, un radical alcoyle de 1 à 6 atomes de carbone, et de préférence un hydrogène ou un méthyle, ainsi que leurs sels obtenus avec des acides organiques ou minéraux physiologiquement acceptables,

caractérisé en ce que :

- soit on prépare le composé racémique en

faisant réagir le racémique d'un composé de formule :

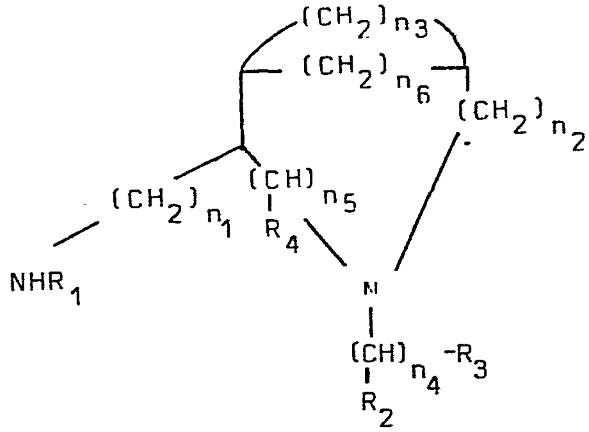

(XXIII)

dans laquelle $R_1$, $R_2$, $R_3$, $R_4$, $n_1$, $n_2$, $n_3$, $n_4$, $n_5$ et $n_6$ ont les significations ci-dessus indiquées sur un sulfochlorure de formule :

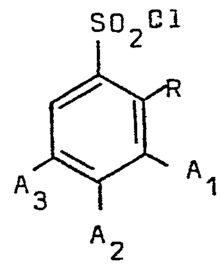

(XV)

dans laquelle R a la signification ci-dessus indiquée et $A_1$, $A_2$, $A_3$ ont les significations respectives de X, Y, Z à l'exception de $NH_2$, suivi d'une éventuelle réduction lorsque l'un au moins des X, Y, Z représente le groupe $NO_2$ ;

- puis on dédouble le racémique obtenu par addition d'acide tartrique optiquement actif, puis alcalinisation ;

- soit on fait réagir directement l'isomère optique de formule :

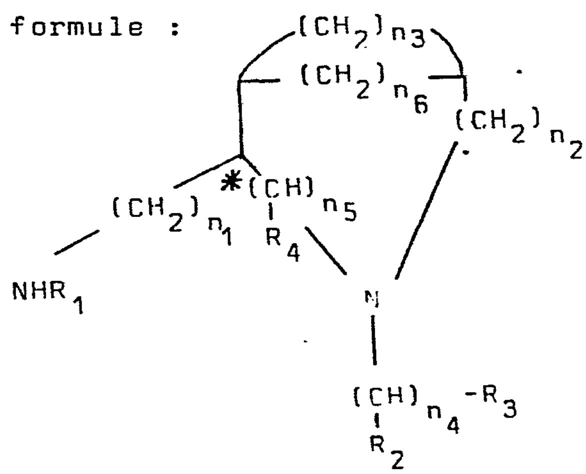

(XXIII)

cet isomère ayant été obtenu par dédoublement à l'acide tartrique du racémique de l'amine correspondante, sur le sulfochlorure de formule :

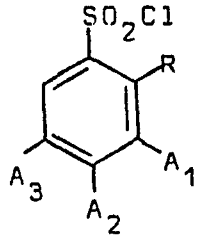

(XV)

dans laquelle R, $A_1$, $A_2$ et $A_3$ ont les significations ci-dessus indiquées.

2. Procédé de préparation de composés lévogyres selon la revendication 1, caractérisé en ce que :

- on a recours à l'amine de formule :

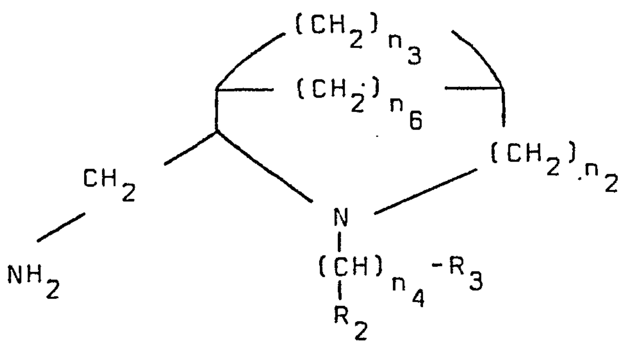

(XXIV)

dans laquelle $n_2$, $n_3$, $n_4$, $n_6$, $R_2$ et $R_3$ ont les significations indiquées à la revendication 1 ;

- cette amine pouvant être obtenue à partir du composé de formule :

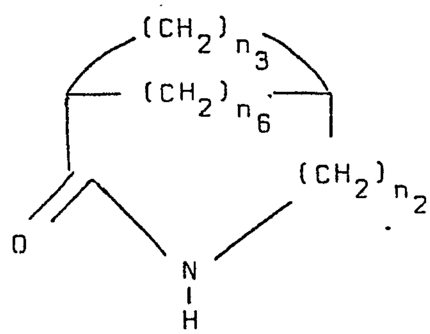

(XXV)

dans laquelle $n_2$, $n_3$, $n_6$ ont les significations indiquées à la revendication 1 ;

- lequel composé de formule (XXV) est soumis à une alcoylation avec un halogénure d'alcoyle de formule :

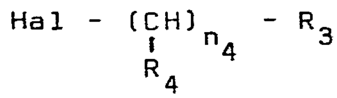

dans laquelle Hal représente un atome d'halogène, en présence d'une base forte telle que l'hydrure de sodium au sein d'un solvant tel que le xylène ; pour donner un composé de formule :

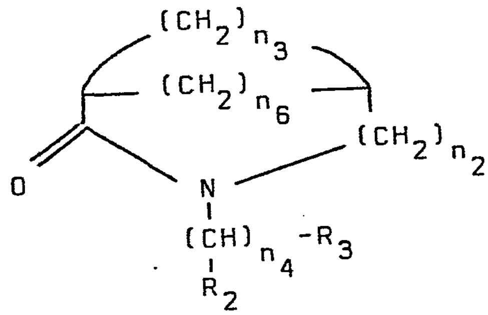

(XXVI)

dans laquelle $n_2$, $n_3$, $n_4$, $n_6$, $R_2$ et $R_3$ ont les significations indiquées à la revendication 1 ;

- lequel composé de formule (XXVI) est ensuite traité par le sulfate de méthyle, le méthylate de sodium et le nitrométhane pour donner le composé de formule (XXVII) suivante :

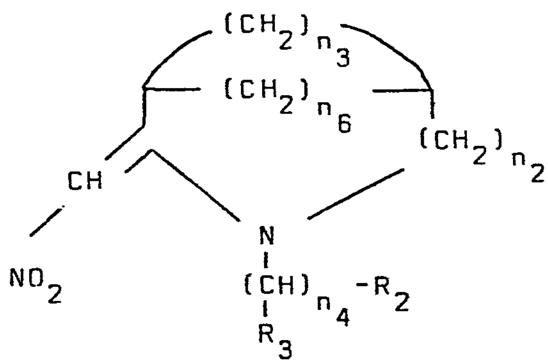

(XXVII)

dans laquelle $n_2$, $n_3$, $n_4$, $n_6$, $R_2$ et $R_3$ ont les significations indiquées ci-dessus ;

- le groupe nitrométhylène du composé de formule (XXVII) étant ensuite réduit à l'aide de nickel de Raney ou d'$AlLiH_4$ pour donner le composé de formule (XXIV).

3. Procédé de préparation de composés lévogyres selon l'une quelconque des revendications 1 ou 2 pour l'obtention des composés lévogyres de formule (IV) :

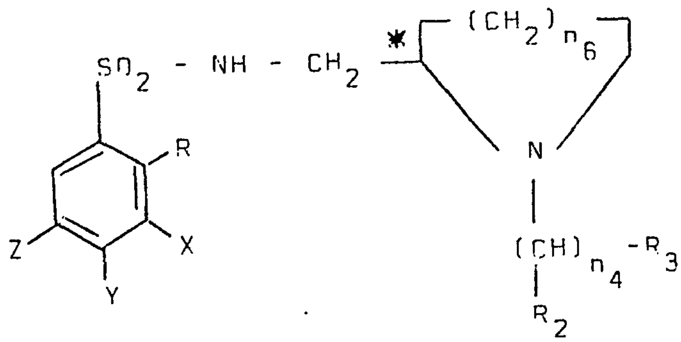

(IV)

dans laquelle $n_6$ vaut 0 à 2, de préférence 2, R,$R_2$,$R_3$,X,Y,Z,$n_4$ ont les significations indiquées aux revendications 1 à 3 ; caractérisé en ce que l'on a recours aux amines de formule (XXVIII) :

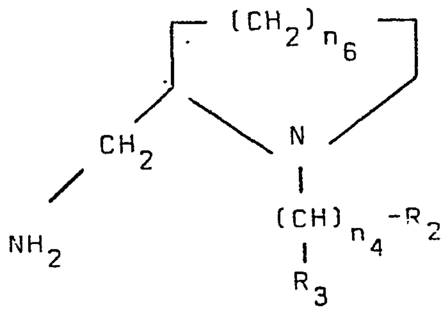

(XXVIII)

obtenues à partir des composés de formule (XXIX) :

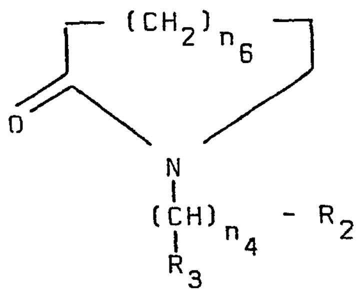

(XXIX)

selon le procédé décrit dans les revendications 1 et 2.

4. Procédé de préparation de composés selon l'une quelconque des revendications 1 à 3 pour l'obtention des composés lévogyres de formule (VIII) :

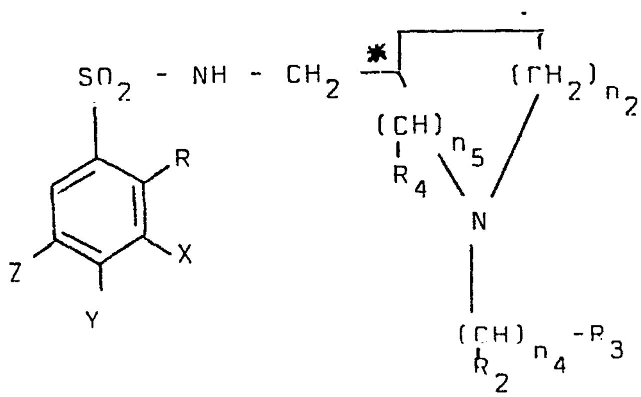

(VIII)

dans laquelle $n_5$ vaut 1 ou 2 et $n_2$ vaut respectivement 1 ou 0, et dans laquelle R, $R_2$, $R_3$, $R_4$, X, Y, Z et $n_4$ ont les significations ci-dessus indiquées ; caractérisé en ce que l'on utilise les amines de formule (XXX) suivante :

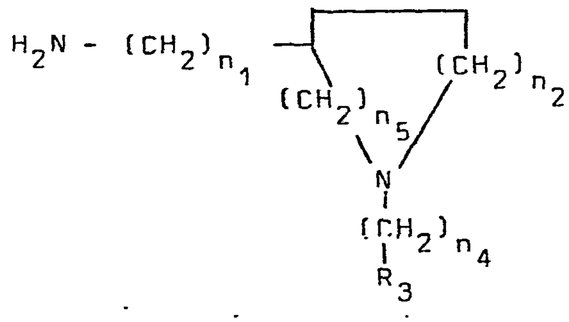

(XXX)

dans laquelle $n_1$, $n_2$, $n_4$, $n_5$ et $R_3$ ont les significations ci-dessus indiquées.

5. Procédé de préparation de composés selon la revendication 4 pour l'obtention de composés lévogyres de formule (VIII) dans laquelle $n_5$ vaut 1 et $n_2$ vaut 1 ; caractérisé en ce qu'on utilise une amine de formule:

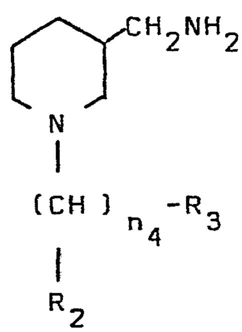

dans laquelle $R_2$,$R_3$ et $n_4$ ont les significations indiquées aux revendications 1 à 4, obtenue par réduction avec $AlLiH_4$ de l'amine de l'acide nipécotique de formule :

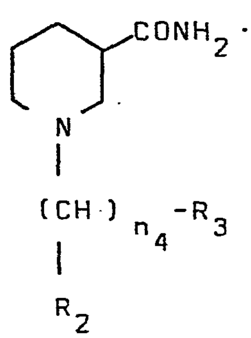

6. Procédé de préparation de composés selon la revendication 4 pour l'obtention des composés lévogyres de formule (VIII) dans laquelle $n_5$ vaut 2 et $n_2$ vaut 0 ; caractérisé en ce qu'on utilise une amine de formule :

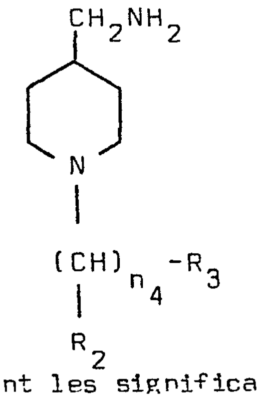

dans laquelle $R_2$, $R_3$ et $n_4$ ont les significations indiquées aux revendications 1 à 4 ; obtenue par réduction avec $AlLiH_4$ de l'amine de l'acide nipécotique de formule :

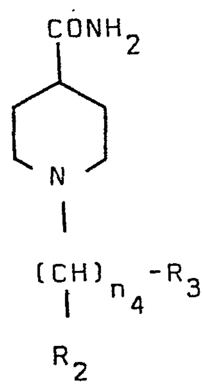

7. Procédé de préparation de composés selon l'une quelconque des revendications 1 à 4 pour l'obtention des composés lévogyres de formule (XII) suivante :

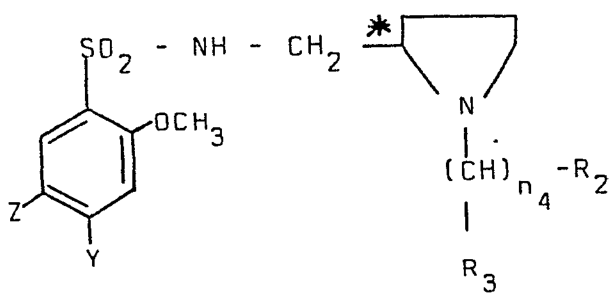

(XII)

dans laquelle $\underset{|\atop R_3}{(CH)}_{n_4}-R_2$ represente l'un des radicaux suivants :

$C_2H_5$, 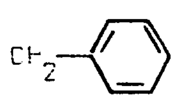 ou 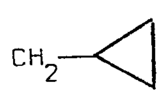

et Y représente $OCH_3$, $NO_2$, $NH_2$ et Z représente Cl, $OCH_3$, $(CH_3)_2CH-SO_2$ ;

caractérisé en ce que :

- l'on a recours à l'amine de formule :

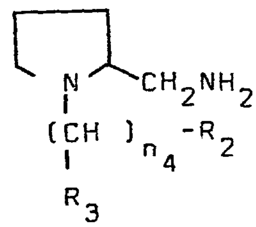

(XXXI)

- laquelle amine peut être obtenue à partir du composé de formule (XXXII) :

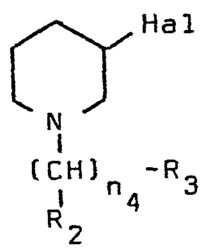

(XXXII)

dans laquelle $R_2$, $R_3$ et $n_4$ ont les significations ci-dessus indiquées, Hal représente un halogène, de préférence le chlore, qui est mise en réaction avec le composé de formule :

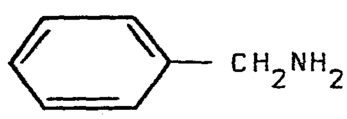

pour donner le composé de formule (XXXIII) :

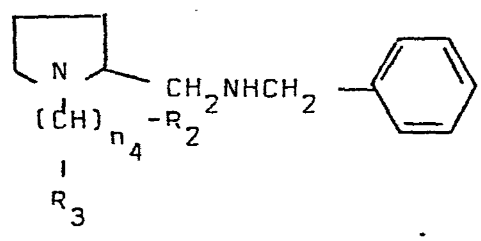

(XXXIII)

- lequel composé de formule (XXXIII) est ensuite soumis à une hydrogénolyse catalytique en présence de charbon palladié pour donner le composé de formule (XXXI).

8. Procédé de préparation de composés selon la revendication 1 pour l'obtention de composés lévogyres

de formule (XXXIV) :

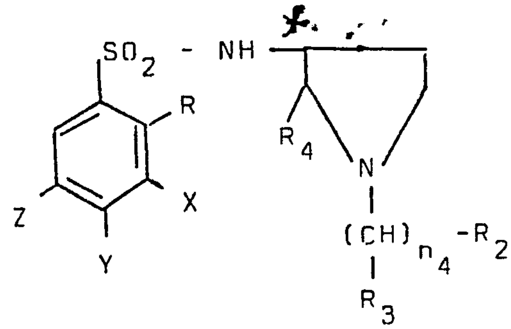

(XXXIV)

dans laquelle X, Y,Z,R,$R_2$, $R_3$ et $R_4$ ont les significations indiquées aux revendications 1 à 7 ; caractérisé en ce qu'on a recours aux pyrrolidines de formule (XXXV) :

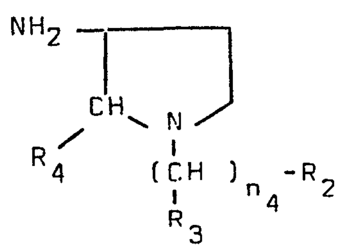

(XXXV)

qui peuvent être préparées par hydrogénation des composés de formule (XXXVI) :

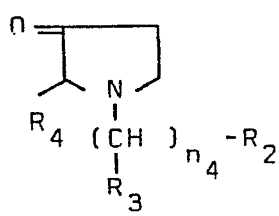

(XXXVI)

9. Procédé de préparation de composés selon l'une quelconque des revendications 1 à 8, caractérisé en ce que le produit obtenu sous forme de chlorhydrate est transformé en la base correspondante ou bien en un sel différent suivi éventuellement d'une réduction lorsque l'un des $A_1$, $A_2$, $R_3$ représente le groupe $NO_2$.

10. Procédé de préparation de composés selon l'une quelconque des revendications 1 à 9, caractérisé en ce que

l'amine est choisie parmi :

- l'aminométhyl-2 éthyl-1 pyrrolidine,
- l'aminométhyl-2 benzyl-1 pyrrolidine,
- l'aminométhyl-2 cyclopropyl-1 pyrrolidine,
- l'aminométhyl-2 éthyl-1 azépine,
- l'aminométhyl-3 benzyl-1 pipéridine,
- l'aminométhyl-3 (chloro-4 benzyl)-1 pipéridine,
- l'aminométhyl-4 benzyl-1 pipéridine,
- l'aminométhyl-4 (chloro-4 benzyl)-1 pipéridine.

11. Procédé de préparation selon l'une quelconque des revendications 4 à 10, caractérisé en ce que le sulfochlorure de formule (XV) est obtenu à partir des arylamines suivantes :

- diméthoxy-2,4 aniline,
- chloro-5 diméthoxy-2,4 aniline,
- bromo-5 diméthoxy-2,4 aniline,
- triméthoxy-2,4,5 aniline,
- diméthoxy-2,4 méthylsulfonyl-5 aniline,
- diméthoxy-2,4 éthylsulfonyl-5 aniline,
- diméthoxy-2,4 propylsulfonyl-5 aniline
- diméthoxy-2,4 isopropylsulfonyl-5 aniline.

Office européen des brevets

# RAPPORT DE RECHERCHE EUROPEENNE

0088849
Numéro de la demande

EP 82 40 0441

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,Y | FR-A-2 386 524 (CHOAY S.A.) *En entier* | 1-7 | C 07 D 207/09<br>C 07 D 211/26<br>C 07 D 223/04<br>A 61 K 31/395 |
| Y | GB-A-2 014 990 (RAVIZZA S.A.) *Résumé* | 1-7 | |
| A | FR-M- 6 895 (BAYER A.G.) *Résumé* | 1,12 | |
| | | | **DOMAINES TECHNIQUES RECHERCHES (Int. Cl. 3)** |
| | | | C 07 D 207/00<br>C 07 D 211/00<br>C 07 D 223/00<br>A 61 K 31/00 |

Le présent rapport de recherche a été établi pour toutes les revendications

| Lieu de la recherche | Date d'achèvement de la recherche | Examinateur |
|---|---|---|
| LA HAYE | 22-11-1982 | MAISONNEUVE J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82